# EUROPEAN PATENT APPLICATION

(11) **EP 4 542 213 A1**
(43) Date of publication of application: **23.04.2025**
(21) Application number: 24206640.5
(22) Date of filing: 15.10.2024
(51) Int. Cl.: G01N 27/327, C12Q 1/00, G01N 27/416, G01N 33/543, G01N 35/00, G16B 40/00

(54) **METHOD OF CREATING LEARNING DATA AND METHOD OF PREDICTING CHARACTERISTIC**

(30) Priority: 17.10.2023 JP 2023179069
(71) Applicant: ARKRAY, Inc., Kyoto-shi, Kyoto 601-8045 (JP)
(72) Inventor: IMANAKA, Norihiro, Kyoto, 602-0008 (JP)
(74) Representative: Dehns

(57) **Abstract**

In a case in which a K value, which is a value related to a concentration of the analyte, is predicted using the prediction model, a control unit of an analysis device acquires a prescribed number of output values indicating a characteristic of the analyte every interval a from a specific time for plural analytes repeatedly a prescribed number of times, and creates learning data by associating a reference K value, which is a K value obtained from a time when a first repetition is executed with the output values of each of the analytes acquired during each repetition.

## Description

### BACKGROUND

### Technical Field

The present invention relates to a method of creating learning data to be used for generating a prediction model that predicts a characteristic of an analyte, and a method of predicting a characteristic.

### Related Art

Japanese National-Phase Publication (JP-A) No. 2020-507773 describes a measurement method of measuring a concentration of glucose using an enzyme electrode.

As a characteristic of an analyte is repeatedly analyzed by an analysis device, the analyte characteristic measurement accuracy may decrease.

In this regard, in a conventional analysis device, for example, in a case in which a characteristic of an analyte is analyzed up to a predetermined number of times, or in a case in which a predetermined time has elapsed from the start of the analysis of the characteristic of the analyte, it is recommended to perform correction work, that is, calibration, on a predicted value.

However, performing calibration in the middle of the analysis work is burdensome for the analyzer.

### SUMMARY

At least preferred embodiments of the invention provide a method of creating learning data to be used for machine learning for generating a prediction model capable of repeatedly predicting a characteristic of an analyte without performing calibration, and a method of predicting a characteristic of an analyte.

According to a first aspect of the invention, there is provided a method of creating learning data in an analysis device including: a reaction cell into which a buffer solution and an analyte are introduced; an electrode provided in the reaction cell, and configured to output a response current having a magnitude corresponding to a concentration of the analyte in the buffer solution in contact with the electrode when a voltage having a predetermined magnitude is applied; and a control unit configured to control a timing at which each of the buffer solution and the analyte is introduced into the reaction cell, and perform a control to acquire the magnitude of the response current output from the electrode as an output value indicating a characteristic of the analyte, the method including: in a case in which the control unit predicts a K value, which is a value related to the concentration of the analyte, the K value being indicated by a difference between an output value Dₜ₁ at time t1 when the control for introducing the analyte into the reaction cell into which the buffer solution has been introduced is performed and an output value Dₜ₂ at time t2 predetermined as a time after the time 11, using a prediction model generated by supervised machine learning, an acquisition step of, for each of a plurality of analytes having different predetermined concentrations, acquiring an output value Dₜ₁ at the time 11, an output value Dₜ₃₊ᵢₐ at time t3+ia (i=0 to n-1, and n is a predetermined integer of 2 or more) at every predetermined interval a within a period after the time t1 and before the time t2, from time t3, which is the start of the period, and an output value Dₜ₂ at the time t2 at least once at a first time, and then repeating the acquisition of the output value Dₜ₃₊ᵢₐ at the time t3+ia for each of the analytes up to a predetermined number of times; a K value calculation step of calculating a K value for each of the analytes as a reference K value from a difference between the output value Dₜ₁ for each of the analytes and the output value Dₜ₂ for each of the analytes acquired at the first time in the acquisition step; and a creation step of creating learning data to be used for supervised machine learning of the prediction model by associating the reference K value of the analyte having the same concentration as the analyte introduced into the reaction cell to acquire each output value Dₜ₃₊ᵢₐ with the output value Dₜ₃₊ᵢₐ of each of the analytes acquired each time the acquisition step is executed.

According to a second aspect of the invention, there is provided a method of predicting a characteristic of an analyte, the method including: an input step of inputting an output value Dₜ₃₊ᵢₐ indicating the characteristic of the analyte at time t3+ia (i=0 to n-1, and n is a predetermined integer of 2 or more), the output value Dₜ₃₊ᵢₐ being acquired at every predetermined interval a within a period after time t1, at which a control is performed to introduce the analyte into a reaction cell, into which a buffer solution and the analyte are introduced, and before time t2, which is predetermined as a time after the time 11, from time t3, which is the start of the period, to a prediction model that predicts a K value, which is a value related to a concentration of the analyte, the model being generated by supervised machine learning using the learning data created by the method of creating learning data according to the first aspect, and optionally including optional features of the first aspect; and a prediction step of predicting a concentration of the analyte from a predicted value of the K value output by the prediction model to which the output value Dₜ₃₊ᵢₐ has been input.

Accordingly, it is possible to create learning data to be used for machine learning for generating a prediction model that repeatedly predicts a characteristic of an analyte without performing calibration.

### BRIEF DESCRIPTION OF THE DRAWINGS

Fig. 1 is a diagram illustrating an example of an external appearance of an analysis device;
Fig. 2 is a diagram illustrating examples of flow paths in the analysis device;
Fig. 3 is a diagram illustrating a principle of measuring a glucose concentration using a glucose sensor;
Fig. 4 is a diagram illustrating a relationship between an output value and a glucose concentration;
Fig. 5 is a diagram illustrating examples of main components in an electrical system of the analysis device including a control unit;
Fig. 6 is a flowchart illustrating an example of a flow of learning data creation processing;
Fig. 7 is a flowchart illustrating an example of a flow of a data creation process in learning data creation example A;
Fig. 8 is an example of a result of evaluating a predicted K value predicted by a prediction model A;
Fig. 9 is a flowchart illustrating an example of a flow of a data creation process in learning data creation example B;
Fig. 10 is a flowchart illustrating an example of a flow of a data creation process in a modification of the learning data creation example B;
Fig. 11 is a diagram illustrating an example of a range in which time t4 is set; and
Fig. 12 is an example of a result of evaluating a predicted K value predicted by a prediction model B.

### DETAILED DESCRIPTION

Hereinafter, the present embodiment will be described with reference to the drawings. The same components and the same processes are denoted by the same reference numerals throughout the drawings, and redundant description will be omitted. The dimensional proportions in the drawings are exaggerated for convenience of description and may be different from actual proportions.

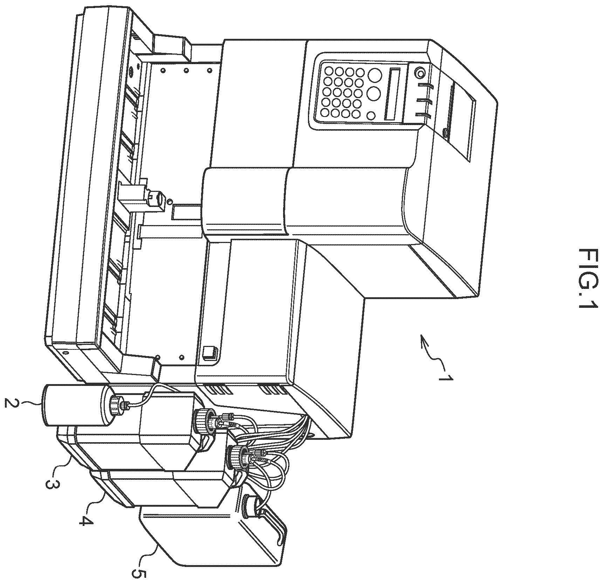

Fig. 1 is a diagram illustrating an example of an external appearance of an analysis device 1 according to the present embodiment. As an example, the analysis device 1 measures a value correlated with a glucose concentration in urine to predict a concentration of glucose contained in the urine, but the analyte is not limited to the urine, and may be another object such as blood. The characteristic of the analyte measured by the analysis device 1 is not limited to the value correlated with the glucose concentration, and may be another characteristic. The value correlated with the glucose concentration will be described in detail below.

As shown in Fig. 1, a standard solution container 2 containing a standard solution, a buffer solution container 3 containing a buffer solution, a cleaning solution container 4 containing a cleaning solution, and a drain container 5 containing an unnecessary solution used by the analysis device 1 are attached to the analysis device 1. The standard solution container 2, the buffer solution container 3, the cleaning solution container 4, and the drain container 5 are connected to the analysis device 1 by tubes.

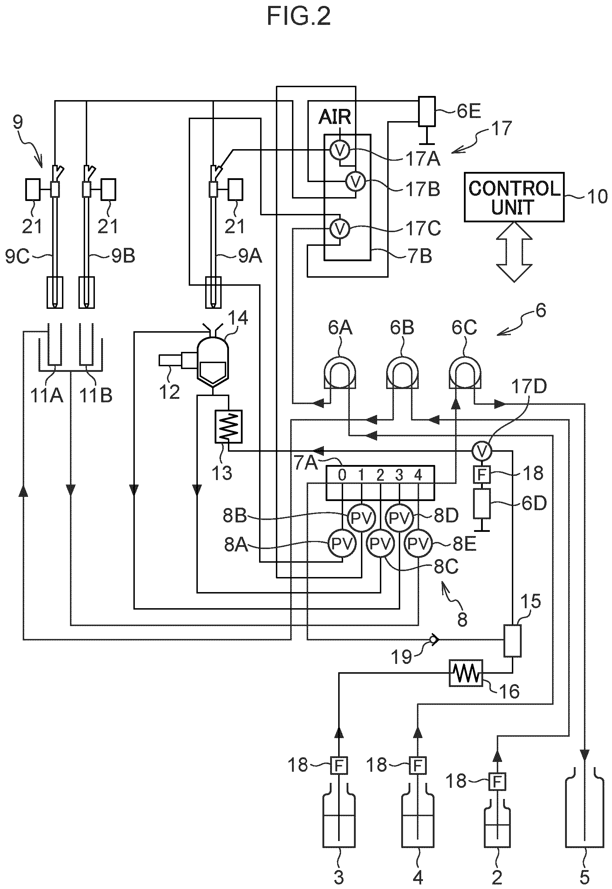

Fig. 2 is a diagram illustrating examples of flow paths for various solutions in the analysis device 1. The analysis device 1 includes a cleaning solution supply pump 6A that sucks up a cleaning solution from the cleaning solution container 4 and supplies the cleaning solution to a predetermined flow path, a standard solution supply pump 6B that sucks up a standard solution from the standard solution container 2 and supplies the standard solution to a predetermined flow path, a buffer solution feeding pump 6D that sucks up a buffer solution from the buffer solution container 3 and supplies the buffer solution to a predetermined flow path, and a sample pump 6E that sucks up a sample, which is an example of an analyte including blood, urine, or the like collected from a subject, from a sample container (not illustrated) and supplies the sample to a predetermined flow path. The analysis device 1 also includes a drain pump 6C that sucks various unnecessary solutions remaining in the flow paths and discharges the unnecessary solutions to the drain container 5. Hereinafter, the cleaning solution supply pump 6A, the standard solution supply pump 6B, the drain pump 6C, the buffer solution feeding pump 6D, and the sample pump 6E may be collectively referred to as "pumps 6".

A filter 18 for removing foreign substances is provided at a designated portion of the flow path. By inserting a nozzle 9A into the sample container and driving the sample pump 6E, the sample is sucked into the nozzle 9A.

A manifold 7B controls a destination to which the flow paths are connected by opening or closing the flow paths using the three-way electromagnetic valves 17A to 17C, and changes the form in which the flow paths are branched.

A flow path to which the sample pump 6E is connected to suck the sample into the nozzle 9A under the control using the three-way electromagnetic valves 17A to 17C is formed in the manifold 7B. In addition, an air suction port and a flow path to which the nozzle 9A is connected to allow air to flow into the nozzle 9A under the control using the three-way electromagnetic valves 17A to 17C are formed in the manifold 7B. When air flows into the nozzle 9A, the sample sucked into the nozzle 9A is discharged from the tip of the nozzle 9A. In addition, a path for discharging an unnecessary sample sucked into the nozzle 9Ato the drain container 5 via the manifold 7A under the control using the three-way electromagnetic valves 17Ato 17C is formed. In addition, a flow path to which the flow path for supplying the cleaning solution is connected to supply the cleaning solution to the tip of the nozzle 9A under the control using the three-way electromagnetic valve 17A to 17C is formed in the manifold 7B.

In addition to the nozzle 9A, the analysis device 1 includes a nozzle 9B and a nozzle 9C through which the solutions are sucked and discharged under the same control as the control for the nozzle 9A. In the example illustrated in Fig. 2, the nozzle 9B is attached at a position corresponding to a cleaning tank 11B containing the cleaning solution, and the nozzle 9C is attached at a position corresponding to a standard solution tank 11A containing the standard solution. Hereinafter, the nozzle 9A, the nozzle 9B, and the nozzle 9C may be collectively referred to as "nozzles 9". There is no restriction on the number of nozzles 9 attached to the analysis device 1. The nozzles 9 can move up, down, left, and right, for example, by an actuator 21.

The manifold 7A is connected to the drain pump 6C. By controlling the opening and closing of the flow paths using pinch valves 8A to 8E, a flow path from which an unnecessary solution is to be sucked is selected from among the plurality of flow paths connected to the manifold 7A, and the unnecessary solution sucked by the drain pump 6C is discharged to the drain container 5. Hereinafter, the pinch valves 8A to 8E may be collectively referred to as "pinch valves 8".

A three-way electromagnetic valve 17D controls the direction in which the buffer solution flows in. The buffer solution supplied to the flow path by the buffer solution feeding pump 6D passes through the three-way electromagnetic valve 17D, and then is introduced into a reaction cell 14 via a heating unit 13.

The heating unit 13 is a device that sets the buffer solution to a predetermined temperature. Hereinafter, the three-way electromagnetic valve 17A to the three-way electromagnetic valve 17D may be collectively referred to as "three-way electromagnetic valves 17".

In addition, a degassing module 15 and a degasser 16 are provided on the flow path for the buffer solution. The degassing module 15 is a member that separates an unnecessary component such as air dissolved in the buffer solution from the buffer solution, and branches a solution containing the unnecessary component into a flow path with a check valve 19 connected to the manifold 7A. The degasser 16 is a member that degasses the buffer solution before the buffer solution enters the buffer solution feeding pump 6D, thereby preventing air bubbles from accumulating in the buffer solution feeding pump 6D and realizing smooth solution feeding.

In such a configuration, the sample sucked into the nozzle 9A is introduced into the reaction cell 14 containing the buffer solution. The reaction cell 14 is provided with a glucose sensor 12, and the glucose sensor 12 measures a concentration of glucose 25 contained in the sample (hereinafter referred to as a "glucose concentration").

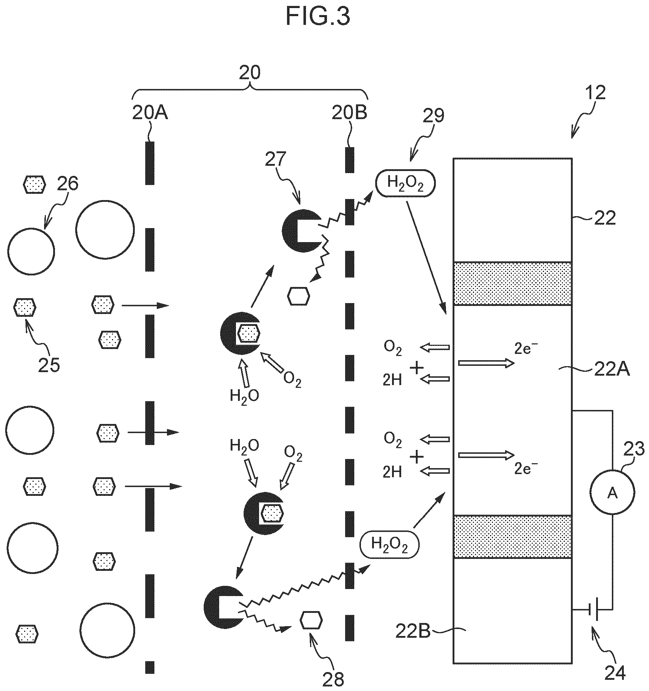

Fig. 3 is a diagram illustrating a principle of measuring a glucose concentration using the glucose sensor 12. The glucose sensor 12 measures a glucose concentration using an enzyme electrode in which a glucose oxidase (GOD)-immobilized enzyme membrane 20 and a hydrogen peroxide electrode 22 are combined.

The GOD-immobilized enzyme membrane 20 is an enzyme membrane in which GOD 27 is wrapped with a polycarbonate membrane 20A and a cellulose acetate membrane 20B. The sample introduced into the reaction cell 14 includes, for example, glucose 25 and protein 26, but the polycarbonate membrane 20A has pores of such a size that only the glucose 25 passes through the polycarbonate membrane 20A while not allowing the protein 26 to pass through the polycarbonate membrane 20A, specifically, pores of about 300 angstroms (30 nanometers). Therefore, only the glucose 25 passes through the polycarbonate membrane 20A. The glucose 25 that has entered the GOD-immobilized enzyme membrane 20 is decomposed into gluconic acid 28 and hydrogen peroxide 29 by the action of GOD 27 as expressed by Formula (1).

C₆H₁₂O₆+O₂+H₂O → C₆H₁₂O₇+H₂O₂ (1)

The cellulose acetate membrane 20B has pores of such a size that only the hydrogen peroxide 29 passes through the cellulose acetate membrane 20B, specifically, pores of from 5 angstroms to 6 angstroms (0.5 to 0.6 nanometers). Therefore, the decomposed hydrogen peroxide 29 passes through the cellulose acetate membrane 20B and reaches the hydrogen peroxide electrode 22. The hydrogen peroxide electrode 22 is an electrode in which platinum is used for an anode 22A and silver is used for a cathode 22B. A voltage source 24 is connected between the anode 22A and the cathode 22B. When a voltage of a predetermined magnitude is applied from the voltage source 24, the hydrogen peroxide 29 having reached the hydrogen peroxide electrode 22 causes an oxidation-reduction reaction by the voltage applied by the voltage source 24, and a current flows between the anode 22A and the cathode 22B.

As described above, since the polycarbonate membrane 20A has holes of such a size that only the hydrogen peroxide 29 passes through the polycarbonate membrane 20A, the polycarbonate membrane 20Ais not affected by the interference reaction caused by the reducing substance. Therefore, a stable current continues to flow between the anode 22A and the cathode 22B. The current flowing between the anode 22A and the cathode 22B indicates a magnitude associated with a glucose concentration in the buffer solution. The current flowing between the anode 22A and the cathode 22B according to the glucose concentration as described above is referred to as a "response current".

The reaction at the anode 22A and the reaction at the cathode 22B are expressed by Formulas (2) and (3), respectively.

H₂O₂ → 2H₂+O₂+2e⁻ (2)

O₂+4H+4e⁻ → 2H₂O (3)

An ammeter 23 is connected between the anode 22A and the cathode 22B, and the response current is subjected to analog to digital (AD) conversion by the ammeter 23, thereby obtaining an output value indicating the magnitude of the response current.

The glucose concentration is calculated from a difference between an output value Dₜ₁ at time t1 when a control is performed to introduce a sample into the reaction cell 14 into which the buffer solution has been introduced and an output value Dₜ₂ at time t2 that is predetermined as a time after the time t1.

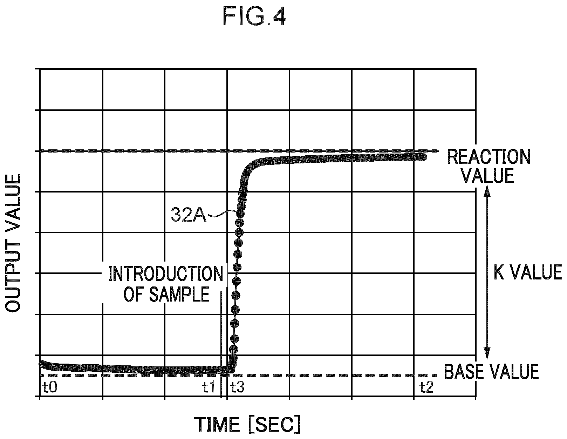

Fig. 4 is a diagram illustrating a relationship between a time that has elapsed from a time at which it is started to measure a response current and an output value. In Fig. 4, the horizontal axis represents a time [sec] that has elapsed from a time at which it is started to measure a response current, and the vertical axis represents an output value. In addition, a curve 32A represents an output value at each time.

The output value Dₜ₁ at the time t1 when the sample is introduced into the reaction cell 14 will be referred to as a base value, and the output value Dₜ₂ at the time t2 will be referred to as a reaction value. The time t2 is a time when the output value changed by introducing the sample into the reaction cell 14 is stabilized. Time t0 represents a time when a predetermined amount of buffer solution is introduced into the reaction cell 14. The output value being stable mean that a variation width (difference) between output values adjacent to one another in time series falls within a range in which it can be considered that no change is recognized between the output values.

In Fig. 4, a difference between the base value and the reaction value is referred to as a K value. The K value is a characteristic value correlated with a glucose concentration. Therefore, the glucose concentration of the sample can be predicted from the K value. Herein, the predicting of the glucose concentration from the K value is referred to as "measuring the glucose concentration". Specifically, a K value of a standard sample whose glucose concentration is known is acquired by the analysis device 1, and a relational expression between the glucose concentration and the K value is acquired in advance. Then, a K value of a sample whose glucose concentration is unknown is measured by the analysis device 1, and the glucose concentration is predicted from the relational expression obtained in advance and the measured value of the K value.

In the above-described example, in principle, it takes time until the time t2 to measure the glucose concentration in the sample. However, the analysis device 1 predicts a K value from a degree of change between output values before the time t2 is reached using a prediction model generated in advance by supervised machine learning, thereby shortening the time required for measuring the glucose concentration.

Specifically, a control unit 10 acquires n (n is an integer of 2 or more) output values Dₜ₃₊ᵢₐ at every interval a, that is, each time the time a elapses, over a predetermined period from time t3, which is the start of the period after the time t1 and before the time t2. Here, "i" is an integer index changing from 0 to (n-1), and the output value Dₜ₃₊ᵢₐ represents an output value at time t3+ia.

The control unit 10 inputs the n output values Dₜ₃₊ᵢₐ acquired after the time t3 to the prediction model to predict a K value. Hereinafter, the n output values Dₜ₃₊ᵢₐ acquired at every interval a from the time t3 may be referred to as "specific output values".

The number n is a value that is predetermined, for example, through an experiment before the glucose concentration is measured. The greater the value of the number n, the more improved the glucose concentration measurement accuracy, but the longer it will take to measure the glucose concentration.

The interval a and the time t3 are also values that are predetermined through, for example, an experiment before the glucose concentration is measured. When the maximum value of the number n is nₘₐₓ, the number n, the interval a, and the time t3 are set such that time t3+a(nₘₐₓ-1) < time t2.

In this way, the analysis device 1 measures a glucose concentration in a sample. However, in the analysis device 1 that uses an enzyme electrode in measuring a glucose concentration, as glucose concentrations are repeatedly measured, an obtained K value deviates from a K value corresponding to an actual glucose concentration, which may reduce the glucose concentration prediction accuracy. This phenomenon is referred to as a "drift phenomenon".

For this reason, the analysis device 1 has a calibration function. The calibration is a process of correcting the deviation between the obtained K value and the K value corresponding to the actual glucose concentration, and has a function of bringing a glucose concentration measured by the analysis device 1 close to the actual glucose concentration even when glucose concentrations are repeatedly measured.

The calibration in the analysis device 1 will be described. The analysis device 1 sucks up a standard solution having a predetermined glucose concentration from the standard solution container 2, introduces the sucked-up standard solution into the reaction cell 14, and predicts a K value of the standard solution by the method described above. When the predicted K value of the standard solution deviates from the standard value of the K value corresponding to the glucose concentration of the standard solution, the analysis device 1 determines a correction coefficient of the predicted K value of the standard solution so that the predicted K value of the standard solution is close to the standard value of the K value corresponding to the glucose concentration of the standard solution. In a subsequent measurement of a glucose concentration, the analysis device 1 corrects a K value using the correction coefficient determined by the calibration, thereby bringing a result of measuring the glucose concentration close to an actual glucose concentration in the sample.

The calibration is generally executed by a user's instruction, for example, when glucose concentrations in samples are measured up to a predetermined number of times, or when a predetermined time has elapsed since the start of measurement of the glucose concentration.

However, the calibration of the analysis device 1 may be performed at a timing that is considered to be particularly necessary by the user, for example, at the start of use of the analysis device 1. As will be described in detail below, since the analysis device 1 has the prediction model that predicts a K value reflecting a correction coefficient, the user can continue to measure a glucose concentration without performing calibration even when glucose concentrations in samples are repeatedly measured.

The control unit 10 controls the timing at which the buffer solution and the sample are introduced into the reaction cell 14 by controlling the pumps 6, the pinch valves 8, the heating unit 13, the three-way electromagnetic valves 17, and the actuator 21, the supply of the cleaning solution and the standard solution to the analysis device 1, the discharge of unnecessary solutions to the drain container 5, the voltage source 24, the calibration, and the acquisition of output values by the ammeter 23.

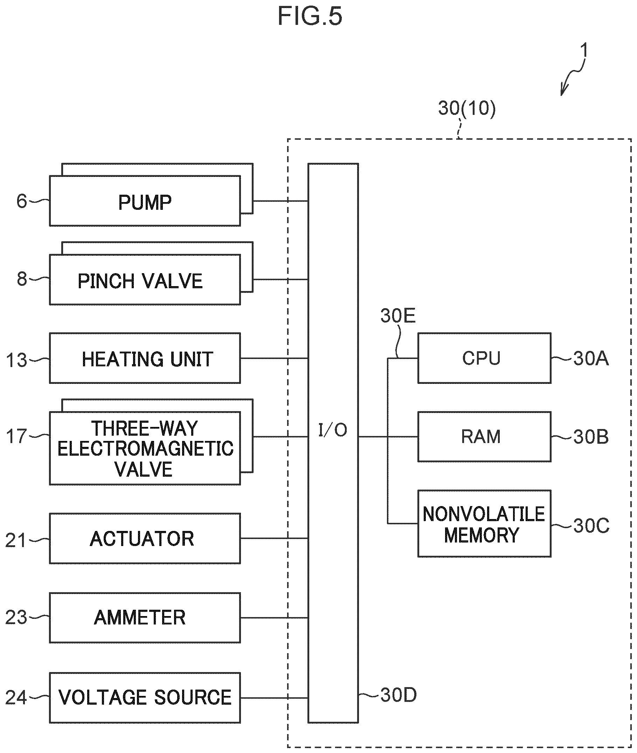

The control unit 10 is configured, for example, using a computer 30. Fig. 5 is a diagram illustrating examples of main components in an electrical system of the analysis device 1 including the control unit 10 configured using the computer 30.

The computer 30 includes a central processing unit (CPU) 30Athat is an example of a processor responsible for processes executed by the control unit 10, a random access memory (RAM) 30B used as a temporary work area of the CPU 30A, a nonvolatile memory 30C, and an input/output interface (I/O) 30D. The CPU 30A, the RAM30B, the nonvolatile memory 30C, and the I/O 30D are connected to each other via a bus 30E.

The nonvolatile memory 30C is an example of a storage device that maintains stored information even if power supplied to the nonvolatile memory 30C is cut off, and for example, a semiconductor memory is used, but a hard disk may be used. The nonvolatile memory 30C stores, for example, control programs that cause the computer 30 to function as the control unit 10, and various parameters to be referred to when a glucose concentration is measured.

Note that the nonvolatile memory 30C is not necessarily built in the computer 30, and may be, for example, a portable storage device detachable from the computer 30, such as a memory card.

Members that operate by receiving instructions from the control unit 10, such as the pumps 6, the pinch valves 8, the heating unit 13, the three-way electromagnetic valves 17, the actuator 21, the ammeter 23, and the voltage source 24, are connected to the I/O 30D, but the members connected to the I/O 30D are not limited thereto. For example, a member corresponding to a function of the analysis device 1, such as a communication unit (not illustrated) that is connected to a communication line to communicate with an external device, can be connected to the I/O 30D.

### <Learning Data Creation Example A>

Next, learning data creation processing for creating learning data to be used for generating a prediction model that predicts a K value will be described in detail.

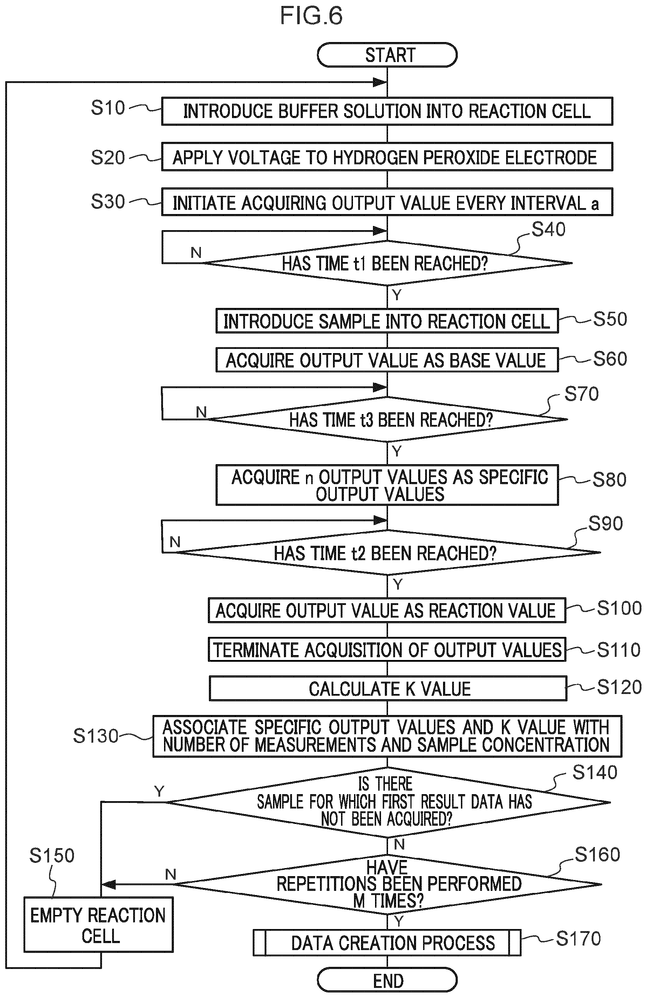

Fig. 6 is a flowchart illustrating an example of a flow of learning data creation processing executed by the control unit 10 when an instruction to create learning data is received from the user. The CPU 30Aof the control unit 10 reads a control program from the nonvolatile memory 30C and executes learning data creation processing.

Prior to the start of the learning data creation processing, a sample set is prepared in advance for the analysis device 1. The sample set refers to a plurality of samples having different predetermined glucose concentrations, and the samples are contained in different sample containers, respectively. Hereinafter, the predetermined glucose concentration in each sample is referred to as "specified concentration".

In step S10, the control unit 10 drives the buffer solution feeding pump 6D in a state where the pinch valve 8C is closed on the flow path connecting the manifold 7A No. 2 and the reaction cell 14, thereby introducing a predetermined amount of buffer solution into the reaction cell 14 provided with the glucose sensor 12 so that the buffer solution comes into contact with the glucose sensor 12.

In step S20, the control unit 10 controls the voltage source 24 to apply a voltage of a predetermined magnitude to the hydrogen peroxide electrode 22 in the glucose sensor 12.

In step S30, the control unit 10 initiates a control to acquire an output value corresponding to a magnitude of a response current measured by the glucose sensor 12 every interval a. The time at which the acquisition of the output value is initiated is defined as time t0. The control unit 10 manages time, for example, using a timer function included in the CPU 30A, but may acquire time information from the outside.

In step S40, while acquiring an output value every interval a, the control unit 10 determines whether or not time t1 has been reached. The time t1 is a time when the output value is stabilized in a state before the sample is introduced into the reaction cell 14. The time t1 is a time determined from a result of an experiment or the like performed in advance before the learning data creation processing is initiated.

Note that the control unit 10 may determine that the time t1 has been reached when the output value is stabilized with reference to a variation width (difference) between output values adjacent to each other in time series. In this case, the time t1 may differ for each learning data creation processing.

In a case in which the time t1 has not been reached, the determination process of step S40 is repeatedly executed, and the control unit 10 continues the process of acquiring an output value every interval a until the time t1 is reached. On the other hand, in a case in which the time t1 has been reached, the process proceeds to step S50.

In step S50, the control unit 10 controls the actuator 21 to move the nozzle 9Ato above a sample container containing one of the unselected samples in the sample set, and then move down the nozzle 9A such that the nozzle 9A enters the sample container. In this state, the control unit 10 drives the sample pump 6E to suck the sample into the nozzle 9A.

Furthermore, the control unit 10 controls the actuator 21 to move the nozzle 9A into which the sample has been sucked to above the reaction cell 14, and then move down the nozzle 9A such that the tip of the nozzle 9A enters the reaction cell 14. In this state, the control unit 10 drives the sample pump 6E to introduce the sample in the nozzle 9A in a predetermined amount into the reaction cell 14. After the sample is introduced into the reaction cell 14, the control unit 10 controls the actuator 21 to move up the nozzle 9A to a position where the tip of the nozzle 9A comes out of the reaction cell 14.

As described above, by introducing the sample into the reaction cell 14 after the output value is stabilized, the output value after the sample is introduced is close to a value that correctly reflects a variation amount of a response current obtained only by an oxidation-reduction reaction of hydrogen peroxide 29 generated from glucose 25 contained in the sample, as compared with that in a case in which the sample is introduced in a state where the output value is not stabilized.

In step S60, after introducing the sample into the reaction cell 14, the control unit 10 stores an output value acquired at a first time as a base value in the nonvolatile memory 30C.

Subsequently, in step S70, while acquiring an output value every interval a, the control unit 10 determines whether or not time t3 has been reached. The time t3 is a time that is predetermined through an experiment before the learning data creation processing is executed, and represents a time that has elapsed from the time t0.

In a case in which it is determined that the time t3 has not been reached, the determination process of step S70 is repeatedly executed, and the control unit 10 continues the process of acquiring an output value every interval a until the time t3 is reached.

On the other hand, in a case in which it is determined that the time t3 has been reached, the process proceeds to step S80.

In step S80, the control unit 10 acquires n output values at every interval a, and stores each of the acquired output values as a specific output value Dₜ₃₊ᵢₐ (i=0 to n-1) in the nonvolatile memory 30C.

In step S90, while acquiring n specific output values Dₜ₃₊ᵢₐ, the control unit 10 determines whether or not time t2 has been reached.

In a case in which the time t2 has not been reached, the determination process of step S90 is repeatedly executed, and the control unit 10 continues the process of acquiring n specific output values Dₜ₃₊ᵢₐ. On the other hand, in a case in which the time t2 has been reached, the process proceeds to step S100.

In step S100, the control unit 10 acquires an output value Dₜ₂ at the time t2, and stores the output value Dₜ₂ as a reaction value in the nonvolatile memory 30C.

In step S110, the control unit 10 controls the voltage source 24 to stop the application of the voltage to the hydrogen peroxide electrode 22 in the glucose sensor 12, and terminate the acquisition of output values.

In step S120, the control unit 10 reads the base value acquired in the process of step S60 and the reaction value acquired in the process of step S100 from the nonvolatile memory 30C, and calculates a K value by subtracting the base value from the reaction value. The control unit 10 stores the calculated K value in the nonvolatile memory 30C.

In step S130, the control unit 10 stores, in the nonvolatile memory 30C, the n specific output values Dₜ₃₊ᵢₐ acquired in the process of step S80 and the K value calculated by the process of step S120 in association with the number of measurements and the specified concentration for the sample sucked into the nozzle 9A in the process of step S50. Data in which the number of measurements for each sample, the specified concentration for the sample, the specific output values Dₜ₃₊ᵢₐ, and the K value are associated with each sample included in the sample set is referred to as "first result data".

In step S140, the control unit 10 determines whether or not there is a sample for which first result data has yet not been acquired in the sample set. In a case in which there is a sample for which first result data has not been acquired, the process proceeds to step S 150.

In order to prepare for acquiring first result data for a next sample included in the sample set, in step S 150, the control unit 10 drives the drain pump 6C in a state where the pinch valve 8C is opened, thereby discharging the buffer solution mixed with the sample from the reaction cell 14, emptying the reaction cell 14, and the process proceeds to step S 10. By doing so, the control unit 10 repeatedly executes the processes of steps S 10 to S 150 until it is determined that there is no sample for which first result data has not been acquired in the determination process of step S 140. As a result, the first result data for each sample included in the sample set is obtained.

In the repeating processes of steps S10 to S150, the order in which the samples are selected in the process of step S50 is the same as that in the repeating processes of the other steps.

On the other hand, in a case in which it is determined in the determination process of step S140 that there is no sample for which first result data has not been acquired in the sample set, the process proceeds to step S160.

In step S 160, it is determined whether or not the acquisition of the first result data for the sample set has been repeated M times. Here, "M" is an index representing the number of repetitions of the acquisition of the first result data on a sample set basis, and is an integer of 2 or more. There is no restriction on the upper limit of the value of the number of repetitions M, and the number of repetitions M is the number of times that is predetermined before the learning data creation processing is executed. The number of repetitions M is stored in advance, for example, in the nonvolatile memory 30C.

In a case in which the acquisition of the first result data for the sample set has not been repeated M times, the process proceeds to step S 150 to empty the reaction cell 14, and then the control unit 10 executes the process of step S 10. By doing so, the control unit 10 repeatedly executes the processes of steps S 10 to S 160 until it is determined in the determination process of step S 160 that the acquisition of the first result data for the sample set has been repeated M times. As a result, M sets of first result data for each sample included in the sample set is obtained.

Hereinafter, in order to distinguish first result data for each sample included in the sample set between sample set units for acquiring first result data, the expression "measurement cycle" may be used. The measurement cycle is a value that increases by one cycle each time the control unit 10 acquires first result data for each sample included in the sample set once.

In other words, a case in which the processes of steps S10 to S 150 is repeated as many times as the number of samples included in the sample set corresponds to one measurement cycle. Therefore, each measurement cycle corresponds to a cycle at which the acquisition of first result data is executed on a sample set basis. In this way, since the number of measurements for each sample included in the sample set is also represented by the measurement cycle, the number of measurements for each sample constituting the first result data is the same value as the measurement cycle.

Note that an aggregate of first result data for each sample included in the sample set, that is, first result data acquired on a sample set basis is simply referred to as "first result data for the sample set".

The control unit 10 acquires M sets of first result data for the sample set by acquiring the first result data for the sample set every measurement cycle.

In a case in which it is determined by the determination process of step S160 that the acquisition of the first result data for the sample set has been repeated M times, the process proceeds to step S170.

In this case, M sets of first result data for the sample set are acquired. Therefore, in step S170, the control unit 10 performs a data creation process of creating learning data for generating a prediction model, and terminates the learning data creation processing illustrated in Fig. 6.

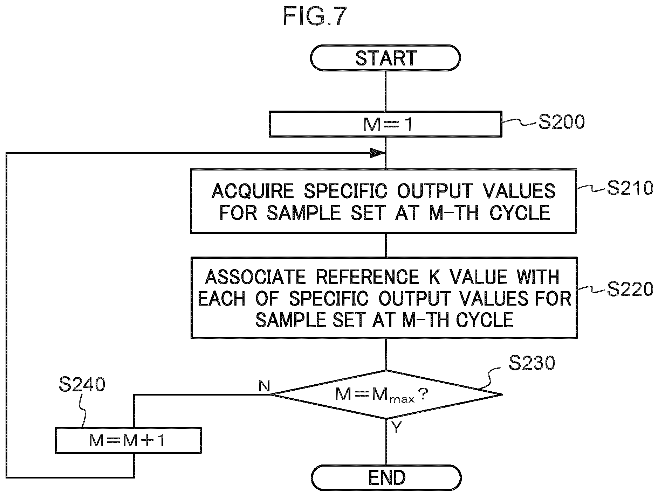

Fig. 7 is a flowchart illustrating an example of a flow of a data creation process in the learning data creation example A.

First, in step S200, the control unit 10 initializes the measurement cycle M to "1".

In step S210, the control unit 10 acquires specific output values Dₜ₃₊ᵢₐ corresponding to respective specified concentrations from the first result data for the sample set at an M-th measurement cycle.

In step S220, the control unit 10 associates a reference K value of a sample having the same specified concentrations as the sample introduced into the reaction cell 14 to acquire each output value Dₜ₃₊ᵢₐ with each of the specific output values Dₜ₃₊ᵢₐ acquired by the process of step S210. The reference K value is a K value calculated by the process of step S120 illustrated in Fig. 6 at the first measurement cycle, and exists for each specified concentration of the sample.

Each data group in which a specific output value Dₜ₃₊ᵢₐ and a reference K value are associated with each other for each specified concentration of the sample set is learning data for the prediction model, with the specific output value Dₜ₃₊ᵢₐ being input data of the learning data and the reference K value being output data of the learning data. The control unit 10 stores each group of created learning data, for example, in the nonvolatile memory 30C.

In step S230, the control unit 10 determines whether or not the measurement cycle M is Mₘₐₓ. Mₘₐₓ represents an upper limit value of the measurement cycle M. In a case in which the measurement cycle M is not the upper limit value Mₘₐₓ, there is first result data for a measurement cycle M at which a specific output value Dₜ₃₊ᵢₐ has yet not been acquired. Therefore, the process proceeds to step S240.

In step S240, the control unit 10 adds "1" to the measurement cycle M to update the measurement cycle M, and proceeds to step S210. By doing so, the control unit 10 repeatedly executes the processes of steps S210 to S240 until it is determined in the determination process of step S230 that the measurement cycle M is Mₘₐₓ. Therefore, learning data in which the specific output value Dₜ₃₊ᵢₐ and the reference K value are associated with each other is created, for each measurement cycle and for each specified concentration of the sample included in the sample set, from the first result data for the sample set at all the measurement cycles M.

On the other hand, in a case in which it is determined in the determination process of step S230 that the measurement cycle M is Mₘₐₓ, since the learning data is created from the first result data for the sample set at all the measurement cycles M, the data creation process illustrated in Fig. 7 ends, and the learning data creation processing illustrated in Fig. 6 also ends with the end of the data creation process.

An example of the learning data created by the learning data creation processing illustrated in Fig. 6 is shown in Table 1. In Table 1, measurement cycles M for samples, specific output values, and reference K values are shown in the order in which the samples are measured from the top of Table 1.

**[Table 1]**

| M | Specific output value | | | | Reference K value |
|---|---|---|---|---|---|
| 1 | D[1:1]ₜ₃ | D[1:1]ₜ₃₊ₐ | ··· | D[1:1]ₜ₃₊ᵢₐ | K1 |
| | D[1:2]ₜ₃ | D[1:2]ₜ₃₊ₐ | ··· | D[1:2]ₜ₃₊ᵢₐ | K2 |
| | | | | | : |
| | D[1:Cₘₐₓ]ₜ₃ | D[1:Cₘₐₓ]ₜ₃₊ₐ | ··· | D[1:Cₘₐₓ]ₜ₃₊ᵢₐ | KCₘₐₓ |
| 2 | D[2:1]ₜ₃ | D[2:1]ₜ₃₊ₐ | ··· | D[2:1]ₜ₃₊ᵢₐ | K1 |
| | D[2:2]ₜ₃ | D[2:2]ₜ₃₊ₐ | ··· | D[2:2]ₜ₃₊ᵢₐ | K2 |
| | | | | | : |
| | D[2:Cₘₐₓ]ₜ₃ | D[2:Cₘₐₓ]ₜ₃₊ₐ | ··· | D[2:Cₘₐₓ]ₜ₃₊ᵢₐ | KCₘₐₓ |
| : | | | | | : |
| Mₘₐₓ | D[Mₘₐₓ:1]ₜ₃ | D[Mₘₐₓ :1]ₜ₃₊ₐ | ··· | D[Mₘₐₓ:1]ₜ₃₊ᵢₐ | K1 |
| | D[Mₘₐₓ:2]ₜ₃ | D[Mₘₐₓ:2]ₜ₃₊ₐ | ··· | D[Mₘₐₓ:2]ₜ₃₊ᵢₐ | K2 |
| | | | | | : |
| | D[Mₘₐₓ:Cₘₐₓ]ₜ₃ | D[Mₘₐₓ:Cᵣₙₐₓ]ₜ₃₊ₐ | ··· | D[Mₘₐₓ:Cₘₐₓ]ₜ₃₊ᵢₐ | KCₘₐₓ |

In Table 1, "M" included in the top item row represents a measurement cycle. Combinations of specific output values and reference K values along the row direction in each measurement cycle M represents learning data created from the first result data for the sample set at the corresponding measurement cycle M.

For convenience of description, a sample included in the sample set and having a different specified concentration from the other samples is distinguished using an index C. The index C is an integer of 1 or more. For example, index C=1 represents a sample having a specified concentration of A [mg/dL] in the sample set, and index C=2 represents a sample having a specified concentration of B [mg/dL] in the sample set. In this way, the C type of sample included in the sample set is distinguished by the value of the index C associated with each of the specified concentrations. In addition, the upper limit value of the index C, that is, the number of samples included in the sample set is represented by Cₘₐₓ.

In order to clearly indicate which specified concentration the sample corresponding to the specific output value Dₜ₃₊ᵢₐ has at which measurement cycle, the specific output value Dₜ₃₊ᵢₐ is distinguished using the index C and the measurement cycle M. The specific output value D[M:C]ₜ₃₊ᵢₐ indicates a specific output value at the time t3+ia of the first result data for the sample having the specified concentration associated with the index C among the first result data for the sample set at the M-th measurement cycle.

In addition, since the reference K value exists for each specified concentration of the sample, the reference K value is distinguished by being expressed as KC (C=1 to Cₘₐₓ). Specifically, the reference K value K1 indicates a reference K value calculated from a sample having a specific specified concentration from which first result data is acquired at the first time at the first measurement cycle, and the reference K value K2 indicates a reference K value calculated from a sample having another specific specified concentration from which first result data is acquired at the second time at the first measurement cycle.

That is, Table 1 shows learning data in which a reference K value KC (C=1 to Cₘₐₓ) is associated with a specific output value D[M:C]ₜ₃₊ᵢₐ (M=1 to Mₘₐₓ, C=1 to Cₘₐₓ, and i=0 to (nₘₐₓ-1)).

By associating the reference K value corresponding to the specified concentration of each sample as output data, learning data excluding the influence of the drift phenomenon is obtained for learning data created from the first result data for the sample set at any measurement cycle M.

Note that, as can be seen from the data creation process illustrated in Fig. 7, in a case in which learning data is created according to the example A of creation of learning data, K values are unnecessary at the second and subsequent measurement cycles M. Therefore, at the second and subsequent measurement cycles M, after a specific output value Dₜ₃₊ᵢₐ is acquired by the process of step S80 in the learning data creation processing illustrated in Fig. 6, the control unit 10 may proceed to step S130 without executing the processes of steps S90 to S120. In this case, the control unit 10 does not calculate a K value for each sample at the second and subsequent measurement cycles M. Therefore, in step S130 at the second and subsequent measurement cycles M, the control unit 10 may associate the n specific output values Dₜ₃₊ᵢₐ acquired by the process of step S80 with the number of measurements and the specified concentration for the sample sucked into the nozzle 9A by the process of step S50, and store the associated data in the nonvolatile memory 30C.

### <Generation of Prediction Model A>

Supervised machine learning was executed by a convolutional neural network (CNN) using the learning data created according to the learning data creation example A to generate a prediction model A. The prediction model A is an example of a prediction model that predicts a K value of a sample from a specific output value Dₜ₃₊ᵢₐ of the sample acquired by the analysis device 1.

Here, the CNN was used for generating the prediction model A as an example, but known supervised machine learning can be used for generating the prediction model A. Specifically, logistic regression analysis, decision tree, random forest, k-nearest neighbor method, support vector machine (SVM), deep learning, or the like may be used instead of the CNN.

### <Evaluation of Prediction Model A>

When a K value of a sample having a known glucose concentration is predicted using the prediction model A, the K value prediction accuracy is evaluated.

Specifically, the learning data creation processing illustrated in Fig. 6 is executed for a sample set having the same combinations of specified concentrations as the sample set in a case in which the learning data is created, and preliminary data having the same data structure as the learning data shown in Table 1 is created. The upper limit value Mₘₐₓ of the measurement cycle M in a case in which the preliminary data was created might be equal to or greater than the upper limit value Mₘₐₓ in a case in which the learning data was created, but was set to a value smaller than the upper limit value Mₘₐₓ in a case in which the learning data was created. As an example, the upper limit value Mₘₐₓ of the measurement cycle M in a case in which the preliminary data was created was set to "140".

Among the preliminary data created in this manner, the preliminary data for the sample having the same specified concentration was extracted every measurement cycle M, and used as data for evaluating the prediction model A. When a specific output value D[M:C]ₜ₃₊ᵢₐ included in the evaluation data was input to the prediction model A, a K value output from the prediction model A, that is, a predicted K value, was acquired, and a K value prediction accuracy of the prediction model A was evaluated from a degree of deviation between the reference K value and the predicted K value included in the same evaluation data.

An example of the data for evaluating the prediction model A is shown in Table 2.

**[Table 2]**

| M | Specific output value | | | | Reference K value |
|---|---|---|---|---|---|
| 1 | D_{L}[1:7]ₜ₃ | D_{L}[1:7]ₜ₃₊ₐ | ··· | D_{L}[1:7]ₜ₃₊ᵢₐ | K_{L}7 |
| 2 | D_{L}[2:7]ₜ₃ | D_{L}[2:7]ₜ₃₊ₐ | ··· | D_{L}[2:7]ₜ₃₊ᵢₐ | K_{L}7 |
| 3 | D_{L}[3:7]ₜ₃ | D_{L}[3:7]ₜ₃₊ₐ | ··· | D_{L}[3:7]ₜ₃₊ᵢₐ | K_{L}7 |
| : | | | | | : |
| 140 | D_{L}[140:7]ₜ₃ | D_{L}[140:7]ₜ₃₊ₐ | ··· | D_{L}[140:7]ₜ₃₊ᵢₐ | K_{L}7 |

In order to distinguish from the specific output value D[M:C]ₜ₃₊ᵢₐ of the learning data, the specific output value of the evaluation data is represented by D_{L}[M:C]ₜ₃₊ᵢₐ. In addition, in order to distinguish from the reference K value KC of the learning data, the reference K value of the evaluation data is represented by K_{L}C. As can be seen from Table 2, evaluation data was created from a sample having a specified concentration corresponding to index C=7.

The deviation rate is used as an index indicating a K value prediction accuracy of the prediction model A. The deviation rate of the predicted K value is represented by Formula (4). Deviation rate [%] of predicted K value = (predicted K value - reference K value)/reference K value × 100

For comparison with the deviation rate of the predicted K value, a deviation rate of a K value of a sample from which the evaluation data is created when the specific output value D_{L}[M:C]ₜ₃₊ᵢₐ of the evaluation data is acquired, that is, a measured K value, is calculated together. The deviation rate of the measured K value is represented by Formula (5). Deviation rate [%] of measured K value = (measured K value - reference K value)/reference K value × 100

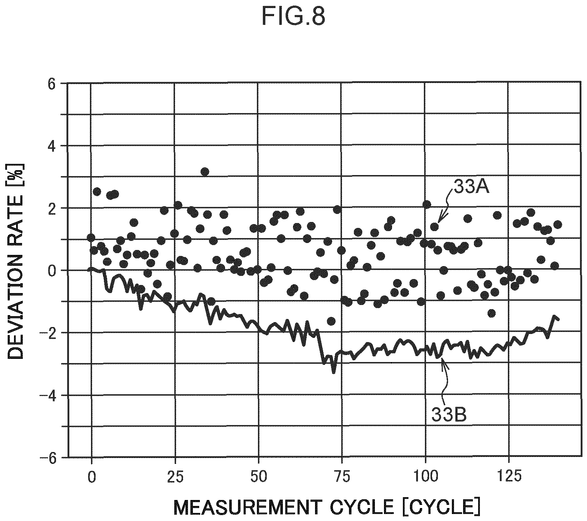

Fig. 8 is an example of an evaluation result indicating deviation rates of predicted K values in the evaluation data shown in Table 2, predicted by the prediction model A, and deviation rates of measured K values in the evaluation data shown in Table 2. In the evaluation result illustrated in Fig. 8, the horizontal axis represents an evaluation data measurement cycle M, and the vertical axis represents a deviation rate. In addition, a graph 33A illustrated in Fig. 8 represents predicted K values, and a graph 33B represents measured K values.

As illustrated in Fig. 8, in the conventional method in which measured K values were repeatedly measured without using the prediction model A and without performing calibration in the middle, the deviation rates tended to increase as the measurement cycle M increased, and deviations of up to 3.5% occurred with respect to the reference K values.

On the other hand, in a case in which K values of samples were predicted using the prediction model A, the predicted K values tended to have deviation rates within a certain range regardless of the measurement cycle M, not showing a tendency toward an increase in deviation rate according to the increase in measurement cycle M.

Specifically, the predicted K values deviated by only about 2.4% with respect to the reference K values. Therefore, even though glucose concentrations are repeatedly measured by the analysis device 1 that predicts K values using the prediction model A, there is no need to perform calibration for the analysis device 1 while the glucose concentrations are repeatedly measured.

That is, even though glucose concentrations of samples are repeatedly measured, the analysis device 1 that measures the glucose concentrations using the prediction model A can suppress errors of the measured glucose concentrations within a certain range.

### <Learning Data Creation Example B>

Next, learning data creation processing for creating learning data to be used for generating a prediction model that predicts a K value by a method different from that in the learning data creation example A will be described in detail.

The learning data creation processing in the learning data creation example B is different from the learning data creation processing in the learning data creation example A illustrated in Fig. 6 in the data creation process of step S170. Since the other processes are the same as those in the learning data creation processing illustrated in Fig. 6, the data creation process performed in step S170 of Fig. 6 will be described below.

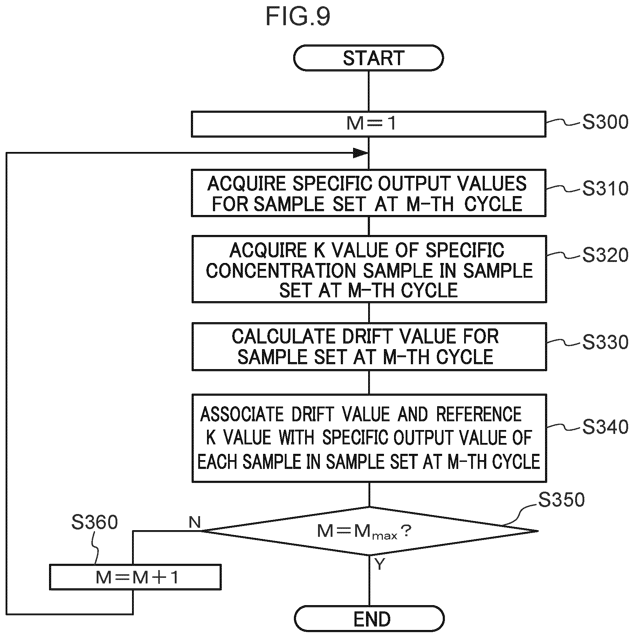

Fig. 9 is a flowchart illustrating an example of a flow of a data creation process in the learning data creation example B. The CPU 30A of the control unit 10 reads a control program from the nonvolatile memory 30C and executes a data creation process.

First, in step S300, the control unit 10 initializes the measurement cycle M to "1".

In step S310, the control unit 10 acquires specific output values Dₜ₃₊ᵢₐ corresponding to respective specified concentrations from the first result data for the sample set at an M-th measurement cycle.

In step S320, the control unit 10 acquires a K value of a sample having a specified concentration specified in advance, among the samples included in the sample set, from the first result data at the M-th measurement cycle. Hereinafter, the sample having the specified concentration specified in advance is referred to as "specific concentration sample". The specific concentration sample is an example of a specific analyte.

The number of specific concentration samples is not necessarily one, and may be plural. In the analysis device 1, since the measurement accuracy may be different depending on the glucose concentration to be measured, a measurement accuracy for a specific glucose concentration may be higher than measurement accuracies for the other glucose concentrations. Therefore, in a case in which the number of specific concentration samples is one, it is preferable to select, for example, a sample of which the glucose concentration estimated from the reference K value is closest to the actual glucose concentration of the sample, as the specific concentration sample, from among the samples included in the sample set. In this case, the glucose concentration measurement accuracy of the analysis device 1 may be improved as compared with that in a case in which another sample is used as the specific concentration sample.

Such a specific concentration sample is predetermined, for example, through an experiment before the learning data creation processing is executed.

In a case in which the number of specific concentration samples is, for example, two, for example, samples of which the glucose concentrations estimated from the reference K values are closest first and second to the actual glucose concentration of the sample, as the specific concentration samples, from among the samples included in the sample set.

In step S330, the control unit 10 calculates, for each specific concentration sample, a drift value indicating a degree of deviation between the reference K value of the specific concentration sample and the K value of the specific concentration sample acquired by the process of step S320.

There is no restriction on the method of calculating the drift value, as long as it indicates a degree of deviation between the reference K value of the specific concentration sample and the K value of the specific concentration sample at the M-th measurement cycle. For example, as shown in Formula (6), the drift value is calculated by a difference between the reference K value of the specific concentration sample and the K value of the specific concentration sample at the M-th measurement cycle. Drift value at M-th cycle = K value of specific concentration sample at M-th measurement cycle - reference K value of specific concentration sample

The drift value may be calculated as (reference K value - K value) by replacing the term on the right side of Formula (6).

In addition, for example, as shown in Formula (7), the drift value may be calculated by a ratio of the K value of the specific concentration sample at the M-th measurement cycle to the reference K value of the specific concentration sample. Drift value at M-th cycle = K value of specific concentration sample at M-th measurement cycle/reference K value of specific concentration sample

The drift value may be calculated as reference K value/K value by replacing the denominator and the numerator on the right side of Formula (7).

In step S340, the control unit 10 associates the drift value calculated by the process of step S330 with the specific output value Dₜ₃₊ᵢₐ of each sample included in the sample set at the M-th measurement cycle. Furthermore, the control unit 10 associates a reference K value of the sample having the same specified concentration as the sample introduced into the reaction cell 14 to acquire each output value Dₜ₃₊ᵢₐ with the specific output value Dₜ₃₊ᵢₐ of each sample included in the sample set at the M-th measurement cycle.

Each data group in which a specific output value Dₜ₃₊ᵢₐ, a drift value, and a reference K value are associated with each other for each specified concentration of the sample set is learning data for the prediction model, with the specific output value Dₜ₃₊ᵢₐ and the drift value being input data of the learning data and the reference K value being output data of the learning data. The control unit 10 stores each group of created learning data, for example, in the nonvolatile memory 30C.

As can be seen from the process of step S340, the same drift value is associated with all the learning data created from the first result data for the sample set at the same measurement cycle M.

In a case in which there are a plurality of specific concentration samples, a plurality of drift values are obtained, and accordingly, the plurality of drift values is associated with each data group of learning data.

In step S350, the control unit 10 determines whether or not the measurement cycle M is Mₘₐₓ. In a case in which the measurement cycle M is not the upper limit value Mₘₐₓ, there is first result data for a measurement cycle M at which a specific output value Dₜ₃₊ᵢₐ has yet not been acquired. Therefore, the process proceeds to step S360.

In step S360, the control unit 10 adds "1" to the measurement cycle M to update the measurement cycle M, and proceeds to step S310. By doing so, the control unit 10 repeatedly executes the processes of steps S310 to S360 until it is determined in the determination process of step S350 that the measurement cycle M is the upper limit value Mₘₐₓ.

Therefore, learning data in which the specific output value Dₜ₃₊ᵢₐ, the drift value, and the reference K value are associated with each other is created, for each measurement cycle M and for each specified concentration of the sample included in the sample set, from the first result data for the sample set at all the measurement cycles M.

On the other hand, in a case in which it is determined in the determination process of step S350 that the measurement cycle M is the upper limit value Mₘₐₓ, since the learning data is created from the first result data for the sample set at all the measurement cycles M, the data creation process illustrated in Fig. 9 ends, and the learning data creation processing illustrated in Fig. 6 also ends with the end of the data creation process.

Table 3 shows an example of learning data created in a case in which the process of step S170 in the learning data creation processing illustrated in Fig. 6 is replaced with the data creation process illustrated in Fig. 9. The learning data shown in Table 3 is learning data in a case in which there is one specific concentration sample. For convenience of description, a drift value created from the first result data for the sample set at the M-th measurement cycle is represented by ΔK(M).

**[Table 3]**

| M | Specific output value | | | | Drift value | Reference K value |
|---|---|---|---|---|---|---|
| 1 | D[1:1]ₜ₃ | D[1:1]ₜ₃₊ₐ | ··· | D[1:1]ₜ₃₊ᵢₐ | ΔK(1) | K1 |
| | D[1:2]ₜ₃ | D[1:2]ₜ₃₊ₐ | ··· | D[1:2]ₜ₃₊ᵢₐ | | K2 |
| | | | | | | : |
| | D[1:Cₘₐₓ]ₜ₃ | D[1:Cₘₐₓ]ₜ₃₊ₐ | ··· | D[1:Cₘₐₓ]ₜ₃₊ᵢₐ | | KCₘₐₓ |
| 2 | D[2:1]ₜ₃ | D[2:1]ₜ₃₊ₐ | ··· | D[2:1]ₜ₃₊ᵢₐ | ΔK(2) | K1 |
| | D[2:2]ₜ₃ | D[2:2]ₜ₃₊ₐ | ··· | D[2:2]ₜ₃₊ᵢₐ | | K2 |
| | | | | | | : |
| | D[2:Cₘₐₓ]ₜ₃ | D[2:Cₘₐₓ]ₜ₃₊ₐ | ··· | D[2:Cₘₐₓ]ₜ₃₊ᵢₐ | | KCₘₐₓ |
| : | | | | | | : |
| Mₘₐₓ | D[Mₘₐₓ:1]ₜ₃ | D[Mₘₐₓ:1]ₜ₃₊ₐ | ··· | D[Mₘₐₓ:1]ₜ₃₊ᵢₐ | ΔK(Mₘₐₓ) | K1 |
| | D[Mₘₐₓ:2]ₜ₃ | D[Mₘₐₓ:2]ₜ₃₊ₐ | ··· | D[Mₘₐₓ:2]ₜ₃₊ᵢₐ | | K2 |
| | | | | | | : |
| | D[Mₘₐₓ:Cₘₐₓ]ₜ₃ | D[Mₘₐₓ:Cₘₐₓ]ₜ₃₊ₐ | ··· | D[Mₘₐₓ:Cₘₐₓ]ₜ₃₊ᵢₐ | | KCₘₐₓ |

That is, Table 3 shows learning data in which a drift value ΔK(M) (M=1 to Mₘₐₓ) and a reference K value KC (C=1 to Cₘₐₓ) are associated with a specific output value D[M:C]ₜ₃₊ᵢₐ (M=1 to Mₘₐₓ, C=1 to Cₘₐₓ, and 1=0 to (nₘₐₓ-1)).

As can be seen from the data creation process illustrated in Fig. 9, in a case in which learning data is created according to the learning data creation example B, a K value of only the specific concentration sample, among the samples included in the sample set, may be calculated at the second and subsequent measurement cycles M. Therefore, at the second and subsequent measurement cycles M, after a specific output value Dₜ₃₊ᵢₐ is acquired by the process of step S80 in the learning data creation processing illustrated in Fig. 6 for a sample other than the specific concentration sample, the control unit 10 may proceed to step S130 without executing the processes of steps S90 to S 120. In this case, the control unit 10 does not associate a K value with a specific output value Dₜ₃₊ᵢₐ in step S130 for a sample whose K value has not been calculated.

That is, in the first result data for the sample set at the second and subsequent measurement cycles M, the K value is set only to the first result data obtained from the specific concentration sample. At the second and subsequent measurement cycles M, by calculating a K value of only a specific concentration sample among the samples included in the sample set, the time for creating learning data can be shortened as compared with that in a case in which a K value of each sample included in the sample set is calculated every measurement cycle M.

### <Modification of Learning Data Creation Example B>

In the learning data creation example B, the same drift value is set to learning data created from the first result data for the sample set at each M-th measurement cycle. That is, in the learning data creation example B, since the same drift value is set to learning data on a measurement cycle M basis, it is necessary to calculate a K value of a specific concentration sample for each measurement cycle M.

Hereinafter, a creation method of creating learning data without calculating a K value of a specific concentration sample for each measurement cycle M will be described.

Therefore, the control unit 10 bundles a plurality of consecutive measurement cycles M from the first measurement cycle, among the measurement cycles M, by the same number. For example, in a case in which the measurement cycle M having the upper limit value Mₘₐₓ of "6" is bundled by two, the control unit 10 divides the measurement cycles M into three bundles of measurement cycles M, that is, a bundle of the first and second measurement cycles M, a bundle of the third and fourth measurement cycles M, and a bundle of the fifth and sixth measurement cycles M. Then, the control unit 10 processes the bundles of measurement cycles M as a new unit of repetitions.

Hereinafter, in order to distinguish a measurement cycle M and a bundle of measurement cycles M, the bundle of measurement cycles M is referred to as a "data set cycle Mₚ". Here, "Mₚ" is an integer of 2 or more. The upper limit value Mₚₘₐₓ of the data set cycle Mₚ is represented by the upper limit value Mₘₐₓ of the measurement cycle M/Q. "Q" is the number of measurement cycles M bundled into one data set cycle Mₚ, and is an integer of 1 or more. The user presets the measurement cycle number Q such that the upper limit value Mₚₘₐₓ of the data set cycle Mₚ becomes an integer with respect to the upper limit value Mₘₐₓ of the measurement cycle M. In a case in which the measurement cycle number Q is "2", two measurement cycles M correspond to one data set cycle Mₚ. Note that, in a case in which the measurement cycle number Q is "1", the data set cycle Mₚ = the measurement cycle M, which corresponds to the method of creating the learning data according to the learning data creation example B described above. Therefore, in order for the data set cycle Mₚ to include a plurality of measurement cycles M, the measurement cycle number Q is set to two or more.

In the learning data creation processing in the present modification, the data creation process executed in step S170 of the learning data creation processing in the learning data creation example A illustrated in Fig. 6 is replaced with a data creation process different from the data creation process illustrated in Fig. 9. Since the other processes are the same as those in the learning data creation processing illustrated in Fig. 6, the data creation process performed in step S170 of Fig. 6 will be described below.

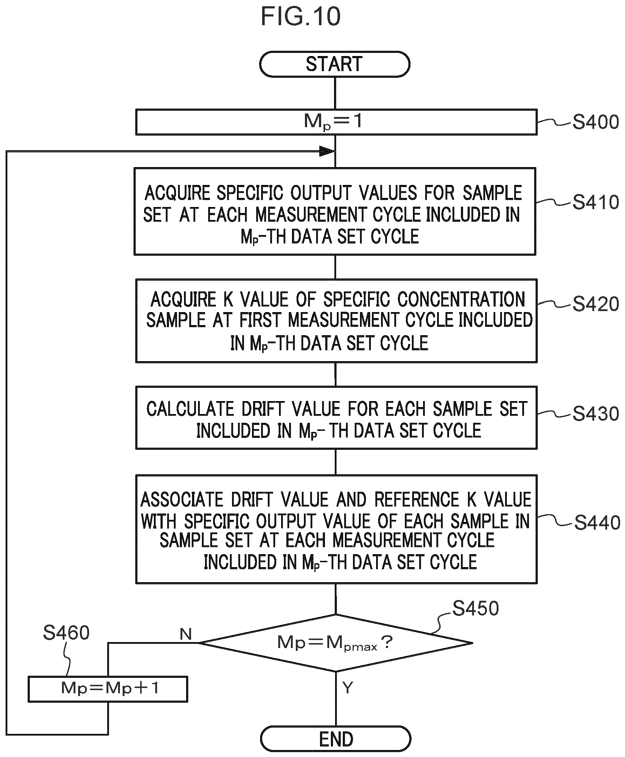

Fig. 10 is a flowchart illustrating an example of a flow of a data creation process in the present modification. The CPU 30A of the control unit 10 reads a control program from the nonvolatile memory 30C and executes a data creation process.

First, in step S400, the control unit 10 initializes the data set cycle Mₚ to "1".

In step S410, the control unit 10 acquires specific output values Dₜ₃₊ᵢₐ corresponding to respective specified concentrations from the first result data for the sample set at each measurement cycle M included in an Mₚ-th data set cycle.

Since one data set cycle Mₚ includes a plurality of measurement cycles M, a K value of a specific concentration sample is calculated a plurality of times within the same data set cycle. Therefore, in step S420, the control unit 10 acquires a K value from the first result data for the specific concentration sample at the first measurement cycle M included in the Mₚ-th data set cycle.

Note that the number of specific concentration samples is not necessarily one, and may be plural. In this case, the control unit 10 acquires, for each specific concentration sample, a K value from the first result data for the specific concentration sample at the first measurement cycle M included in the Mₚ-th data set cycle.

In step S430, the control unit 10 calculates a drift value for each specific concentration sample.

In step S440, the control unit 10 associates the drift value calculated by the process of step S430 with the specific output value Dₜ₃₊ᵢₐ of each sample in the sample set at each measurement cycle M included in the Mₚ-th data set cycle. Further, the control unit 10 associates a reference K value of the sample having the same specified concentration as the sample introduced into the reaction cell 14 to acquire each output value Dₜ₃₊ᵢₐ with the specific output value Dₜ₃₊ᵢₐ of each sample in the sample set at each measurement cycle M included in the Mₚ-th data set cycle.

Each data group in which a specific output value Dₜ₃₊ᵢₐ, a drift value, and a reference K value are associated with each other for each specified concentration of the sample set at each measurement cycle M included in the Mₚ-th data set cycle is learning data for the prediction model, with the specific output value Dₜ₃₊ᵢₐ and the drift value being input data of the learning data and the reference K value being output data of the learning data. The control unit 10 stores each group of created learning data, for example, in the nonvolatile memory 30C.

As can be seen from the process of step S440, the same drift value is set to all the learning data created from the first result data for the sample set at each measurement cycle M included in the same data set cycle Mₚ.

In a case in which there are a plurality of specific concentration samples, a plurality of drift values are obtained, and accordingly, the plurality of drift values is set to each data group of learning data.

In step S450, the control unit 10 determines whether or not the data set cycle Mₚ is the upper limit value Mₚₘₐₓ. In a case in which the data set cycle Mₚ is not the upper limit value Mₚₘₐₓ, there is first result data for a measurement cycle M at which a specific output value Dₜ₃₊ᵢₐ has yet not been acquired. Therefore, the process proceeds to step S460.

In step S460, the control unit 10 adds "1" to the data set cycle Mₚ to update the data set cycle Mₚ, and proceeds to step S410. By doing so, the control unit 10 repeatedly executes the processes of steps S410 to S460 until it is determined in the determination process of step S450 that the data set cycle Mₚ is the upper limit value Mₚₘₐₓ. Therefore, learning data in which the specific output value Dₜ₃₊ᵢₐ, the drift value, and the reference K value are associated with each other is created, for each data set cycle Mₚ and for each specified concentration of the sample included in the sample set at each measurement cycle M included in the data set cycle Mₚ, from the first result data for the sample set at each measurement cycle M included in all the data set cycles Mₚ.

On the other hand, in a case in which it is determined in the determination process of step S450 that the data set cycle Mₚ is the upper limit value Mₚₘₐₓ, since the learning data is created from the first result data for the sample set at each measurement cycle M included in all the data set cycles Mₚ, the data creation process illustrated in Fig. 10 ends, and the learning data creation processing illustrated in Fig. 6 also ends with the end of the data creation process.

Table 4 shows an example of learning data created in a case in which the process of step S170 in the learning data creation processing illustrated in Fig. 6 is replaced with the data creation process illustrated in Fig. 10. The learning data shown in Table 4 is learning data in a case in which there is one specific concentration sample.

**[Table 4]**

| Mₚ | M | Specific output value | | | | Drift value | Reference K value |
|---|---|---|---|---|---|---|---|
| 1 | 1 | D[1:1]ₜ₃ | D[1:1]ₜ₃₊ₐ | ··· | D[1:1]ₜ₃₊ᵢₐ | ΔK(1) | K1 |
| | | D[1:2]ₜ₃ | D[1:2]ₜ₃₊ₐ | ··· | D[1:2]ₜ₃₊ᵢₐ | | K2 |
| | | : | | | | | : |
| | | D[1:Cₘₐₓ]ₜ₃ | D[1:Cₘₐₓ]ₜ₃₊ₐ | ··· | D[1:Cₘₐₓ]ₜ₃₊ᵢₐ | | KCₘₐₓ |
| | 2 | D[2:1]ₜ₃ | D[2:1]ₜ₃₊ₐ | ··· | D[2:1]ₜ₃₊ᵢₐ | | K1 |
| | | D[2:2]ₜ₃ | D[2:2]ₜ₃₊ₐ | ··· | D[2:2]ₜ₃₊ᵢₐ | | K2 |
| | | : | | | | | : |
| | | D[2:Cₘₐₓ]ₜ₃ | D[2:Cₘₐₓ]ₜ₃₊ₐ | ··· | D[2:Cₘₐₓ]ₜ₃₊ᵢₐ | | KCₘₐₓ |
| : | : | : | | | | : | : |
| Mₚₘₐₓ | Mₘₐₓ -1 | D[Mₘₐₓ-1:1]ₜ₃ | D[Mₘₐₓ-1:1]ₜ₃₊ₐ | ··· | D[Mₘₐₓ-1:1]ₜ₃₊ᵢₐ | ΔK(Mₘₐₓ-1) | K1 |
| | | D[Mₘₐₓ-1:2]ₜ₃ | D[Mₘₐₓ-1:2]ₜ₃₊ₐ | ··· | D[Mₘₐₓ-1:2]ₜ₃₊ᵢₐ | | K2 |
| | | : | | | | | : |
| | | D[Mₘₐₓ-1:Cₘₐₓ]ₜ₃ | D[Mₘₐₓ-1:Cₘₐₓ]ₜ₃₊ₐ | ··· | D[Mₘₐₓ-1:Cₘₐₓ]ₜ₃₊ᵢₐ | | KCₘₐₓ |
| | Mₘₐₓ | D[Mₘₐₓ:1]ₜ₃ | D[Mₘₐₓ:1]ₜ₃₊ₐ | ··· | D[Mₘₐₓ:1]ₜ₃₊ᵢₐ | | K1 |
| | | D[Mₘₐₓ:2]ₜ₃ | D[Mₘₐₓ:2]ₜ₃₊ₐ | ··· | D[Mₘₐₓ:2]ₜ₃₊ᵢₐ | | K2 |
| | | : | | | | | : |
| | | D[Mₘₐₓ:Cₘₐₓ]ₜ₃ | D[Mₘₐₓ:Cₘₐₓ]ₜ₃₊ₐ | ··· | D[Mₘₐₓ:Lₘₐₓ]ₜ₃₊ᵢₐ | | KCₘₐₓ |

That is, Table 4 shows learning data in which a drift value ΔK(1+Q(Mₚ-1)) (Mₚ=1 to Mₚₘₐₓ) and a reference K value KC (C=1 to Cₘₐₓ) are associated with a specific output value D[M:C]ₜ₃₊ᵢₐ (M=1 to Mₘₐₓ, C=1 to Cₘₐₓ, and 1=0 to (nₘₐₓ-1)).

As can be seen from the data creation process illustrated in Fig. 10, in a case in which learning data is created according to the present modification, it is only required to calculate a K value of each sample included in the sample set at the first measurement cycle M included in the first data set cycle Mₚ, and a K value of only a specific concentration sample among the samples included in the sample set at the first measurement cycle M for each data set cycle Mₚ included in the second and subsequent data set cycles Mₚ.

Therefore, at a measurement cycle M that is the second or subsequent measurement cycle M and that does not correspond to the 1+Q(Mₚ-1)-th cycle, after a specific output value Dₜ₃₊ᵢₐ is acquired by the process of step S80 in the learning data creation processing illustrated in Fig. 6 for each sample included in the sample set, the process may proceed to step S130 without executing the processes of steps S90 to S120.

In addition, at a measurement cycle M that is the second or subsequent measurement cycle M and that corresponds to the 1+Q(Mₚ-1)-th cycle, after a specific output value Dₜ₃₊ᵢₐ is acquired by the process of step S80 in the learning data creation processing illustrated in Fig. 6 for a sample other than the specific concentration sample, the control unit 10 may proceed to step S130 without executing the processes of steps S90 to S120.

In this case, the control unit 10 does not associate a K value with a specific output value Dₜ₃₊ᵢₐ in step S130 for a sample whose K value has not been calculated.

In this way, according to the present modification, it is only required to calculate a K value of a specific concentration sample at each first measurement cycle M included in each data set cycle Mₚ, rather than calculating a K value at each measurement cycle M, which shortens the time for creating the learning data as compared with that in the learning data creation example B.

In the learning data creation example B and the modification of the learning data creation example B described above, the difference between the reference K value and the K value of the specific concentration sample is used as the drift value, but a value other than the difference between the reference K value and the K value may be used as the drift value.

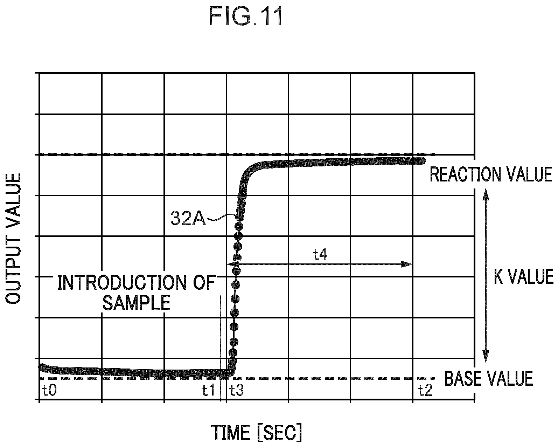

For example, in addition to the acquisition of the specific output value Dₜ₃₊ᵢₐ in the process of step S80 of the learning data creation processing illustrated in Fig. 6, the control unit 10 acquires an output value Dₜ₄ at time t4 which is a time after the time t3 and before the time t2 as illustrated in Fig. 11.

Then, in the process of S120 of Fig. 6, instead of calculating a K value, the control unit 10 calculates a base value, that is, a value of (Dₜ₄-Dₜ₁), which is a difference between the output value Dₜ₁ and the output value Dₜ₄, as an output difference value.

In the following description of each process included in the learning data creation processing, the reference K value is read as an output difference value (hereinafter referred to as "reference output difference value") calculated by the process of step S120 illustrated in Fig. 6 at the first measurement cycle. In addition, the K value is read as an output difference value calculated by the process of step S120 illustrated in Fig. 6 at each measurement cycle M. As a result, as the drift value, values expressed as Formulas (8) and (9) are used instead of the values expressed as Formulas (6) and (7). Drift value at data set cycle Mp = output difference value of specific concentration sample at first measurement cycle M included in data set cycle Mp - reference output difference value of specific concentration sample Drift value at data set cycle Mp = output difference value of specific concentration sample at first measurement cycle M included in data set cycle Mp/reference output difference value of specific concentration sample

As described above, the drift value may be calculated as (reference output difference value - output difference value) by replacing the term on the right side of Formula (8), and the drift value may be calculated as reference output difference value/output difference value by replacing the denominator and the numerator on the right side of Formula (9).

The time t4 is preset by the user, and is stored, for example, in the nonvolatile memory 30C. In a case in which the time t4 is set to the time t2, the output difference value becomes the K value, and the reference output difference value becomes reference K value.

### <Generation of Prediction Model B>

Supervised machine learning was executed by a CNN using the learning data of Table 4 created according to the modification of the learning data creation example B to generate a prediction model B. The prediction model B is an example of a prediction model that predicts a K value of a sample from a specific output value Dₜ₃₊ᵢₐ and a drift value of the sample acquired by the analysis device 1.

Although the CNN was used for generating the prediction model B, known supervised machine learning can be used for generating the prediction model B similarly to the prediction model A. Specifically, logistic regression analysis, decision tree, random forest, k-nearest neighbor method, SVM, deep learning, or the like may be used instead of the CNN.

It goes without saying that the prediction model B may be created using the learning data of Table 3 created according to the learning data creation example B.

### <Evaluation of Prediction Model B>

In a case in which a K value of a sample having a known glucose concentration is predicted using the prediction model B generated by performing supervised machine learning using the learning data shown in Table 4, a K value prediction accuracy is evaluated.

Specifically, the learning data creation processing illustrated in Fig. 6 is executed, while the process of step S170 is replaced with the data creation process illustrated in Fig. 10, for a sample set having the same combinations of specified concentrations as the sample set in a case in which the learning data is created, and preliminary data having the same data structure as the learning data shown in Table 4 is created. The upper limit value Mₘₐₓ of the measurement cycle M in a case in which the preliminary data was created might be equal to or greater than the upper limit value Mₘₐₓ in a case in which the learning data was created, but was set to a value smaller than the upper limit value Mₘₐₓ in a case in which the learning data was created. As an example, the upper limit value Mₘₐₓ of the measurement cycle M in a case in which the preliminary data was created was set to "140". That is, the upper limit value Mₚₘₐₓ of the data set cycle Mₚ is "70".

Among the preliminary data created in this manner, the preliminary data for the sample having the same specified concentration was extracted every measurement cycle M, and used as data for evaluating the prediction model B. When a specific output value D_{L}[M:C]ₜ₃₊ᵢₐ and the drift value ΔK(M) included in the evaluation data was input to the prediction model B, a predicted K value output from the prediction model B was acquired, and a K value prediction accuracy of the prediction model B was evaluated from a degree of deviation between the reference K value and the predicted K value included in the same evaluation data.

An example of the data for evaluating the prediction model B is shown in Table 5. As can be seen from Table 5, evaluation data was created from a sample having a specified concentration corresponding to index C=7. The measurement cycle number Q was Q=2.

**[Table 5]**

| M | Specific output value | | | | Drift value | Reference K value |
|---|---|---|---|---|---|---|
| 1 | D_{L}[1:7]ₜ₃ | D_{L}[1:7]ₜ₃₊ₐ | ··· | D_{L}[1:7]ₜ₃₊ᵢₐ | ΔK_{L}(1) | K_{L}7 |
| 2 | D_{L}[2:7]ₜ₃ | D_{L}[2:7]ₜ₃₊ₐ | ··· | D_{L}[2:7]ₜ₃₊ᵢₐ | ΔK_{L}(1) | K_{L}7 |
| 3 | D_{L}[3:7]ₜ₃ | D_{L}[3:7]ₜ₃₊ₐ | ··· | D_{L}[3:7]ₜ₃₊ᵢₐ | ΔK_{L}(2) | K_{L}7 |
| 4 | D_{L}[4:7]ₜ₃ | D_{L}[4:7]ₜ₃₊ₐ | ··· | D_{L}[4:7]ₜ₃₊ᵢₐ | ΔK_{L}(2) | K_{L}7 |
| : | : | | | | : | : |
| 139 | D_{L}[139:7]ₜ₃ | D_{L}[139:7]ₜ₃₊ₐ | ··· | D_{L}[139:7]ₜ₃₊ᵢₐ | ΔK_{L}(139) | K_{L}7 |
| 140 | D_{L}[140:7]ₜ₃ | D_{L}[140:7]ₜ₃₊ₐ | ··· | D_{L}[140:7]ₜ₃₊ᵢₐ | ΔK_{L}(139) | K_{L}7 |

In order to evaluate a K value prediction accuracy, deviation rates expressed as Formulas (4) and (5) were calculated in the prediction model B as well.

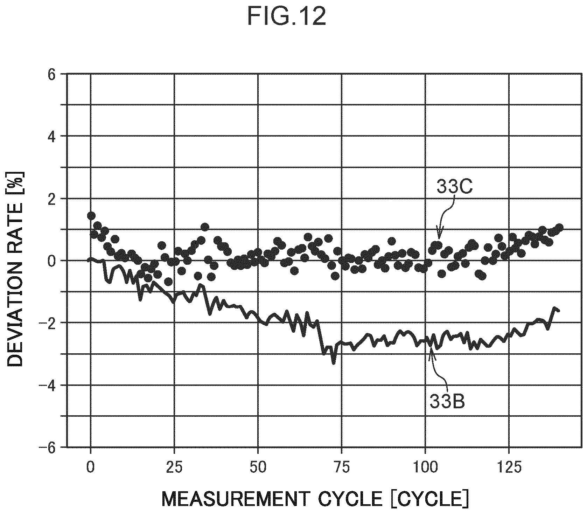

Fig. 12 is an example of an evaluation result indicating deviation rates of predicted K values in the evaluation data shown in Table 5, predicted by the prediction model B, and deviation rates of measured K values in the evaluation data shown in Table 5. In the evaluation result illustrated in Fig. 12, the horizontal axis represents an evaluation data measurement cycle M, and the vertical axis represents a deviation rate. In addition, a graph 33C illustrated in Fig. 12 represents predicted K values, and a graph 33B represents measured K values.

As illustrated in Fig. 12, in the conventional method in which measured K values were repeatedly measured without using the prediction model B and without performing calibration in the middle, deviations of up to 3.5% occurred with respect to the reference K values. On the other hand, in a case in which K values of samples were predicted using the prediction model B, the predicted K values tended to have deviation rates within a certain range regardless of the measurement cycle M, not showing a tendency toward an increase in deviation rate according to the increase in measurement cycle M. Specifically, the predicted K values deviated by only about 1.05% with respect to the reference K values. Therefore, even though glucose concentrations are repeatedly measured by the analysis device 1 that predicts K values using the prediction model B, there is no need to perform calibration for the analysis device 1 while the glucose concentrations are repeatedly measured.

That is, even though glucose concentrations of samples are repeatedly measured, the analysis device 1 that measures the glucose concentrations using the prediction model B can suppress errors of the measured glucose concentrations within a certain range.

Although one mode in which learning data is created by the prediction model used in the analysis device 1 has been described using the embodiment, the mode of creation of learning data disclosed herein is exemplary, and the mode of creation of learning data is not limited to the scope set forth in the embodiment. Various modifications or improvements can be made to the embodiment without departing from the scope of the invention, and the modifications or improvements also fall within the technical scope of the invention, as defined by the appended claims.

For example, the internal processing order in the learning data creation processing illustrated in Fig. 6 and the internal processing order in the data creation process illustrated in each of Figs. 7, 9, and 10 may be modified without departing from the scope of the invention.

In the embodiment, a mode in which the learning data creation processing and the data creation process are realized by software has been described as an example. However, processing equivalent to the flowchart of the learning data creation processing illustrated in Fig. 6 and the flowchart of the data creation process illustrated in each of Figs. 7, 9, and 10 may be processed by hardware. In this case, the processing speed can be increased as compared to that in a case in which each process is realized by software.

In the embodiment, the processor refers to a processor in a broad sense, including a general-purpose processor (e.g., a CPU 30A) or a dedicated processor (e.g., a graphics processing unit (GPU), an application specific integrated circuit (ASIC), a field programmable gate array (FPGA), or a programmable logic device).

The processor operation in the embodiment may be performed not only by one processor but also by a plurality of processors existing at physically separated positions in cooperation. In addition, the order in which the operations are performed by the processor is not limited to the order described in the embodiment, and may be appropriately changed.

It has been described as an example in the embodiment that the control program is stored in advance in the nonvolatile memory 30C. However, the destination in which the control program is stored is not limited to the nonvolatile memory 30C. The control program can also be provided in a recorded form in a storage medium readable by the computer 30.

For example, the control program may be provided in a recorded form on an optical disk such as a compact disk read only memory (CD-ROM), a digital versatile disk read only memory (DVD-ROM), or a Blu-ray disk.

Furthermore, the control program may also be provided in a recorded form on a portable semiconductor memory such as a universal serial bus (USB) memory or a memory card.

The nonvolatile memory 30C, the CD-ROM, the DVD-ROM, the Blu-ray disk, the USB, and the memory card are examples of non-transitory storage media.

Further, the analysis device 1 may download the control program from an external device connected to a communication line through a communication unit, and store the downloaded control program in the nonvolatile memory 30C of the analysis device 1. In this case, the CPU 30A of the analysis device 1 reads the control program downloaded from the external device from the nonvolatile memory 30C and executes the learning data creation processing and the data creation process.

The following clauses set out features of the invention which may not presently be claimed in this application, but which may form the basis for future amendment or a divisional application.
(Clause 1) A method of creating learning data in an analysis device including:
   a reaction cell into which a buffer solution and an analyte are introduced;
   an electrode provided in the reaction cell, and configured to output a response current having a magnitude corresponding to a concentration of the analyte in the buffer solution in contact with the electrode when a voltage having a predetermined magnitude is applied; and
   a control unit configured to control a timing at which each of the buffer solution and the analyte is introduced into the reaction cell, and perform a control to acquire the magnitude of the response current output from the electrode as an output value indicating a characteristic of the analyte, the method including:
      in a case in which the control unit predicts a K value, which is a value related to the concentration of the analyte, the K value being indicated by a difference between an output value Dₜ₁ at time t1 when the control for introducing the analyte into the reaction cell into which the buffer solution has been introduced is performed and an output value Dₜ₂ at time t2 predetermined as a time after the time t1, using a prediction model generated by supervised machine learning,
      an acquisition step of, for each of a plurality of analytes having different predetermined concentrations, acquiring an output value Dₜ₁ at the time t1, an output value Dₜ₃₊ᵢₐ at time t3+ia (i=0 to n-1, and n is a predetermined integer of 2 or more) at every predetermined interval a within a period after the time t1 and before the time t2, from time t3, which is the start of the period, and an output value Dₜ₂ at the time t2 at least once at a first time, and then repeating the acquisition of the output value Dₜ₃₊ᵢₐ at the time t3+ia for each of the analytes up to a predetermined number of times;
      a K value calculation step of calculating a K value for each of the analytes as a reference K value from a difference between the output value Dₜ₁ for each of the analytes and the output value Dₜ₂ for each of the analytes acquired at the first time in the acquisition step; and
      a creation step of creating learning data to be used for supervised machine learning of the prediction model by associating the reference K value of the analyte having the same concentration as the analyte introduced into the reaction cell to acquire each output value Dₜ₃₊ᵢₐ with the output value Dₜ₃₊ᵢₐ of each of the analytes acquired each time the acquisition step is executed.
(Clause 2) The method of creating learning data according to clause 1, further including:
   a calculation step of acquiring an output value Dₜ₄ at time t4, which is a time after the time t3 and before the time t2, for each of the analytes each time the acquisition step is executed, and acquiring an output value Dₜ₁ at the time t1 for each of the analytes each time the acquisition step is executed at the second and subsequent times, and
   for a specific analyte having a specific concentration specified in advance among the analytes, calculating a difference between the output value Dₜ₁ and the output value Dₜ₄ of the specific analyte acquired each time the acquisition step is executed as an output difference value of the specific analyte each time the acquisition step is executed; and
   a drift value calculation step of calculating a drift value each time the acquisition step is executed, the drift value indicating a degree of deviation between the output difference value of the specific analyte at the first time when the acquisition step is executed and the output difference value of the specific analyte each time the acquisition step is executed,
   in which in the creation step, the learning data to be used for the supervised machine learning of the prediction model is created by associating the reference K value of the analyte having the same concentration as the analyte introduced into the reaction cell to acquire each output value Dₜ₃₊ᵢₐ, as output data, with input data acquired each time the acquisition step is executed, the input data including each output value Dₜ₃₊ᵢₐ for each of the analytes each time the acquisition step is executed and the drift value at an execution time that is the same as each execution time when each output value Dₜ₃₊ᵢₐ is acquired.
(Clause 3) The method of creating learning data according to clause 2, in which
   the time t4 is set to the time t2.
(Clause 4) The method of creating learning data according to clause 2 or 3, in which
   the specific analyte is an analyte of which a concentration estimated from the reference K value for each of the analytes is closest to an actual concentration of the analyte among the analytes.
(Clause 5) The method of creating learning data according to clause 2 or 3, in which
   the specific analyte is a plurality of analytes having different predetermined concentrations among the analytes,
   in the drift value calculation step, the drift value for each of the specific analytes is calculated each time the acquisition step is executed, and
   in the creation step, the learning data to be used for the supervised machine learning of the prediction model is created by associating the reference K value of the analyte having the same concentration as the analyte introduced into the reaction cell to acquire each output value Dₜ₃₊ᵢₐ, as output data, with input data acquired each time the acquisition step is executed, the input data including each output value Dₜ₃₊ᵢₐ for each of the analytes each time the acquisition step is executed and a plurality of drift values at an execution time that is the same as each execution time when each output value Dₜ₃₊ᵢₐ is acquired.
(Clause 6) The method of creating learning data according to any one of clauses 2 to 5, in which
   in the drift value calculation step, in a case in which a bundle of a plurality of consecutive times when the acquisition step is executed is treated as a new unit of times when the acquisition step is executed to include a plurality of output difference values of the specific analyte having the same concentration at each new execution time, at least one drift value of the specific analyte is calculated using the output difference value calculated from a difference between the output value Dₜ₁ and the output value Dₜ₄ acquired in the acquisition step performed for the first time at each new execution time, and
   in the creation step, the learning data is created by associating at least one drift value calculated from a new execution time that is the same as the new execution time when the output value Dₜ₃₊ᵢₐ is obtained with each output value Dₜ₃₊ᵢₐ constituting learning data obtained from each execution time constituting the new execution time at which the drift value is calculated.
(Clause 7) The method of creating learning data according to any one of clauses 2 to 6, in which
   the degree of deviation is indicated by a difference between the output difference value of the specific analyte at the first time when the acquisition step is executed and the output difference value of the specific analyte each time the acquisition step is executed, or a ratio of the output difference value of the specific analyte each time the acquisition step is executed to the output difference value of the specific analyte at the first time when the acquisition step is executed.
(Clause 8) A method of predicting a characteristic of an analyte, the method including:
   an input step of inputting an output value Dₜ₃₊ᵢₐ indicating the characteristic of the analyte at time t3+ia (i=0 to n-1, and n is a predetermined integer of 2 or more), the output value Dₜ₃₊ᵢₐ being acquired at every predetermined interval a within a period after time t1, at which a control is performed to introduce the analyte into a reaction cell, into which a buffer solution and the analyte are introduced, and before time t2, which is predetermined as a time after the time t1, from time t3, which is the start of the period, to a prediction model that predicts a K value, which is a value related to a concentration of the analyte, the model being generated by supervised machine learning using the learning data created by the method of creating learning data according to clause 1; and
   a prediction step of predicting a concentration of the analyte from a predicted value of the K value output by the prediction model to which the output value Dₜ₃₊ᵢₐ has been input.
(Clause 9) A method of predicting a characteristic of an analyte, the method including:
   in a case in which a concentration of the analyte is predicted when the analyte is repeatedly introduced into a reaction cell, into which a buffer solution and the analyte are introduced, using a prediction model that predicts a K value, which is a value related to the concentration of the analyte, the model being generated by supervised machine learning using the learning data created by the method of creating learning data according to clause 2,
   an input step of inputting, to the prediction model, an output value Dₜ₃₊ᵢₐ indicating the characteristic of the analyte at time t3+ia (i=0 to n-1, and n is a predetermined integer of 2 or more), the output value Dₜ₃₊ᵢₐ being acquired at every predetermined interval a within a period after time t1, at which a control is performed to introduce the analyte into the reaction cell, and before time t2, which is predetermined as a time after the time t1, from time t3, which is the start of the period, and a drift value indicating a degree of deviation between an output difference value calculated from a difference between an output value Dₜ₁ at the time t1 and an output value Dₜ₄ at time t4, which is a time after the time t3 and before the time t2, the output value Dₜ₁ and the output value Dₜ₄ being obtained from a specific analyte having a specific concentration specified in advance in the same number of times as the number of times the analyte is introduced into the reaction cell when the specific analyte is repeatedly introduced into the reaction cell, and an output difference value obtained when the specific analyte is introduced into the reaction cell for the first time; and
   a prediction step of predicting a concentration of the analyte from a predicted value of the K value output by the prediction model to which the output value Dₜ₃₊ᵢₐ and the drift value have been input.

According to clause 1, there is an advantageous effect in that it is possible to create learning data to be used for machine learning for generating a prediction model that repeatedly predicts a characteristic of an analyte without performing calibration.

According to clause 2, there is an advantageous effect in that the K value prediction accuracy is improved as compared with that in a case in which a K value is predicted using a prediction model for which machine learning is performed based on learning data that does not include a drift value.

According to clause 3, there is an advantageous effect in that a drift value can be calculated from a K value of a specific analyte at each execution time.

According to clause 4, there is an advantageous effect in that the analyte concentration measurement accuracy is improved as compared with that in a case in which a specific analyte is selected without considering a measurement accuracy for each analyte concentration in the analysis device.

According to clause 5, there is an advantageous effect in that the analyte concentration measurement accuracy is improved as compared with that in a case in which a concentration of an analyte is measured using the prediction model generated from the learning data created while the specific analyte is limited to one analyte having a specific concentration.

According to clause 6, there is an advantageous effect in that the time for creating learning data can be shortened as compared with that in a case in which an output difference value of the specific analyte is calculated each time the acquisition step is executed.

According to clause 7, there is an advantageous effect in that a method of calculating a degree of deviation between an output difference value of the specific analyte at the first time when the acquisition step is executed and an output difference value of the specific analyte each time the acquisition step is executed can be selected from among a plurality of calculation methods according to the characteristic of the specific analyte.

According to clauses 8 and 9, there is an advantageous effect in that a characteristic of an analyte can be repeatedly measured without performing calibration.

## Claims

1. A method of creating learning data
in an analysis device (1) including:
a reaction cell (14) into which a buffer solution and an analyte are introduced;
an electrode (22) provided in the reaction cell, and configured to output a response current having a magnitude corresponding to a concentration of the analyte in the buffer solution in contact with the electrode when a voltage having a predetermined magnitude is applied; and
a control unit (10) configured to control a timing at which each of the buffer solution and the analyte is introduced into the reaction cell (14), and perform a control to acquire the magnitude of the response current output from the electrode (22) as an output value indicating a characteristic of the analyte, the method comprising:
in a case in which the control unit (10) predicts a K value, which is a value related to the concentration of the analyte, the K value being indicated by a difference between an output value Dₜ₁ at time t1 when the control for introducing the analyte into the reaction cell into which the buffer solution has been introduced is performed and an output value Dₜ₂ at time t2 predetermined as a time after the time t1, using a prediction model generated by supervised machine learning,
an acquisition step of, for each of a plurality of analytes having different predetermined concentrations, acquiring an output value Dₜ₁ at the time t1, an output value Dₜ₃₊ᵢₐ at time t3+ia, where i=0 to n-1, and n is a predetermined integer of 2 or more, at every predetermined interval a within a period after the time t1 and before the time t2, from time t3, which is the start of the period, and an output value Dₜ₂ at the time t2 at least once at a first time, and then repeating the acquisition of the output value Dₜ₃₊ᵢₐ at the time t3+ia for each of the analytes up to a predetermined number of times;
a K value calculation step of calculating a K value for each of the analytes as a reference K value from a difference between the output value Dₜ₁ for each of the analytes and the output value Dₜ₂ for each of the analytes acquired at the first time in the acquisition step; and
a creation step of creating learning data to be used for supervised machine learning of the prediction model by associating the reference K value of the analyte having the same concentration as the analyte introduced into the reaction cell to acquire each output value Dₜ₃₊ᵢₐ with the output value Dₜ₃₊ᵢₐ of each of the analytes acquired each time the acquisition step is executed.

2. The method of creating learning data according to claim 1, further comprising:
a calculation step of acquiring an output value Dₜ₄ at time t4, which is a time after the time t3 and before the time t2, for each of the analytes each time the acquisition step is executed, and acquiring an output value Dₜ₁ at the time t1 for each of the analytes each time the acquisition step is executed at the second and subsequent times, and
for a specific analyte having a specific concentration specified in advance among the analytes, calculating a difference between the output value Dₜ₁ and the output value Dₜ₄ of the specific analyte acquired each time the acquisition step is executed as an output difference value of the specific analyte each time the acquisition step is executed; and
a drift value calculation step of calculating a drift value each time the acquisition step is executed, the drift value indicating a degree of deviation between the output difference value of the specific analyte at the first time when the acquisition step is executed and the output difference value of the specific analyte each time the acquisition step is executed,
wherein in the creation step, the learning data to be used for the supervised machine learning of the prediction model is created by associating the reference K value of the analyte having the same concentration as the analyte introduced into the reaction cell to acquire each output value Dₜ₃₊ᵢₐ, as output data, with input data acquired each time the acquisition step is executed, the input data including each output value Dₜ₃₊ᵢₐ for each of the analytes each time the acquisition step is executed and the drift value at an execution time that is the same as each execution time when each output value Dₜ₃₊ᵢₐ is acquired.

3. The method of creating learning data according to claim 2, wherein
the time t4 is set to the time t2.

4. The method of creating learning data according to claim 2 or 3, wherein
the specific analyte is an analyte of which a concentration estimated from the reference K value for each of the analytes is closest to an actual concentration of the analyte among the analytes.

5. The method of creating learning data according to claim 2 or 3, wherein
the specific analyte is a plurality of analytes having different predetermined concentrations among the analytes,
in the drift value calculation step, the drift value for each of the specific analytes is calculated each time the acquisition step is executed, and
in the creation step, the learning data to be used for the supervised machine learning of the prediction model is created by associating the reference K value of the analyte having the same concentration as the analyte introduced into the reaction cell to acquire each output value Dₜ₃₊ᵢₐ, as output data, with input data acquired each time the acquisition step is executed, the input data including each output value Dₜ₃₊ᵢₐ for each of the analytes each time the acquisition step is executed and a plurality of drift values at an execution time that is the same as each execution time when each output value Dₜ₃₊ᵢₐ is acquired.

6. The method of creating learning data according to any of claims 2 to 5, wherein
in the drift value calculation step, in a case in which a bundle of a plurality of consecutive times when the acquisition step is executed is treated as a new unit of times when the acquisition step is executed to include a plurality of output difference values of the specific analyte having the same concentration at each new execution time, at least one drift value of the specific analyte is calculated using the output difference value calculated from a difference between the output value Dₜ₁ and the output value Dₜ₄ acquired in the acquisition step performed for the first time at each new execution time, and
in the creation step, the learning data is created by associating at least one drift value calculated from a new execution time that is the same as the new execution time when the output value Dₜ₃₊ᵢₐ is obtained with each output value Dₜ₃₊ᵢₐ constituting learning data obtained from each execution time constituting the new execution time at which the drift value is calculated.

7. The method of creating learning data according to any of claims 2 to 6, wherein
the degree of deviation is indicated by a difference between the output difference value of the specific analyte at the first time when the acquisition step is executed and the output difference value of the specific analyte each time the acquisition step is executed, or a ratio of the output difference value of the specific analyte each time the acquisition step is executed to the output difference value of the specific analyte at the first time when the acquisition step is executed.

8. A method of predicting a characteristic of an analyte, the method comprising:
an input step of inputting an output value Dₜ₃₊ᵢₐ indicating the characteristic of the analyte at time t3+ia (i=0 to n-1, and n is a predetermined integer of 2 or more), the output value Dₜ₃₊ᵢₐ being acquired at every predetermined interval a within a period after time t1, at which a control is performed to introduce the analyte into a reaction cell, into which a buffer solution and the analyte are introduced, and before time t2, which is predetermined as a time after the time t1, from time t3, which is the start of the period, to a prediction model that predicts a K value, which is a value related to a concentration of the analyte, the model being generated by supervised machine learning using the learning data created by the method of creating learning data according to claim 1; and
a prediction step of predicting a concentration of the analyte from a predicted value of the K value output by the prediction model to which the output value Dₜ₃₊ᵢₐ has been input.

9. A method of predicting a characteristic of an analyte, the method comprising:
in a case in which a concentration of the analyte is predicted when the analyte is repeatedly introduced into a reaction cell, into which a buffer solution and the analyte are introduced, using a prediction model that predicts a K value, which is a value related to the concentration of the analyte, the model being generated by supervised machine learning using the learning data created by the method of creating learning data according to any of claims 2 to 7,
an input step of inputting, to the prediction model, an output value Dₜ₃₊ᵢₐ indicating the characteristic of the analyte at time t3+ia (i=0 to n-1, and n is a predetermined integer of 2 or more), the output value Dₜ₃₊ᵢₐ being acquired at every predetermined interval a within a period after time t1, at which a control is performed to introduce the analyte into the reaction cell, and before time t2, which is predetermined as a time after the time t1, from time t3, which is the start of the period, and a drift value indicating a degree of deviation between an output difference value calculated from a difference between an output value Dₜ₁ at the time t1 and an output value Dₜ₄ at time t4, which is a time after the time t3 and before the time t2, the output value Dₜ₁ and the output value Dₜ₄ being obtained from a specific analyte having a specific concentration specified in advance in the same number of times as the number of times the analyte is introduced into the reaction cell when the specific analyte is repeatedly introduced into the reaction cell, and an output difference value obtained when the specific analyte is introduced into the reaction cell for the first time; and
a prediction step of predicting a concentration of the analyte from a predicted value of the K value output by the prediction model to which the output value Dₜ₃₊ᵢₐ and the drift value have been input.
